# EUROPÄISCHE PATENTANMELDUNG

(11) **EP 1 731 036 A1**
(43) Veröffentlichungstag der Anmeldung: **13.12.2006**
(21) Anmeldenummer: 06114975.3
(22) Anmeldetag: 17.10.2002
(51) Int. Cl.: A01N 31/02, A61K 31/045

(54) **Synergistische Mischungen von 1,2-Alkandiolen**

(30) Priorität: 19.02.2002 DE 10206759
(62) Teilanmeldung aus: 02785229.2
(71) Anmelder: Symrise GmbH & Co. KG, 37603 Holzminden (DE)
(72) Erfinder: Schmaus, Gerhard, 37671, Höxter-Bosseborn (DE); Lange, Sabine, 37603, Holzminden (DE); Joppe, Holger, 37586, Dassel (DE)
(74) Vertreter: Eisenführ, Speiser & Partner

(57) **Zusammenfassung**

Beschrieben wird die Verwendung von Mischungen aus zwei, drei oder mehr unverzweigten 1,2-Alkandiolen, deren Kettenlängen (i) unterschiedlich sind und (ii) jeweils im Bereich von 5 bis 10 C-Atomen liegen, als antimikrobielle Wirkstoffe. Die Mischungen besitzen im Vergleich mit den reinen 1,2-Alkandiolen eine synergistisch verstärkte Wirkung.

## Beschreibung

Die vorliegende Erfindung betrifft das Gebiet der antimikrobiellen Wirkstoffe und insbesondere antimikrobielle Wirkstoffe, in denen ein unverzweigtes (geradkettiges) 1,2-Alkandiol in einer antimikrobiell wirksamen Menge vorliegt.

In der kosmetischen und pharmazeutischen sowie in der Lebensmittelindustrie besteht ein beständiger Bedarf an Mitteln mit antimikrobiellen Eigenschaften, insbesondere zur Konservierung ansonsten verderbücher Produkte (wie z.B. Kosmetika, pharmazeutische Produkte oder Lebensmittel), aber auch zur unmittelbaren kosmetischen oder therapeutischen Behandlung von Mikroorganismen, welche einen nachteiligen Einfluss auf den menschlichen oder tierischen Körper besitzen können. Exemplarisch hingewiesen sei auf Mikroorganismen, die Körpergeruch, Akne, Mykosen oder dergleichen verursachen können.

Zwar werden in den angesprochenen technischen Bereichen bereits eine Vielzahl antimikrobieller Wirkstoffe eingesetzt, doch wird weiterhin nach Alternativen gesucht, um gezielte Spezialbehandlungen durchführen und/oder Nebenwirkungen reduzieren zu können. Bei der Suche nach alternativen antimikrobiell und insbesondere konservierend wirkenden Mitteln ist dabei allerdings zu beachten, dass die im kosmetischen, pharmazeutischen und/oder Nahrungsmittelbereich verwendeten Substanzen
- toxikologisch unbedenklich,
- gut hautverträglich,
- stabil (insbesondere in den üblichen kosmetischen und/oder pharmazeutischen Formutierungen),
- weitgehend und vorzugsweise vollständig geruchlos sowie
- preiswert herstellbar (d.h. unter Einsatz von Standardverfahren und/oder ausgehend von Standardprekursoren)
sein müssen.

Die Suche nach geeigneten (Wirk-)Substanzen, die eine oder mehrere der genannten Eigenschaften in ausreichendem Maße besitzen, ist dem Fachmann dadurch erschwert, dass keine klare Abhängigkeit zwischen der chemischen Struktur einer Substanz einerseits und ihrer biologischen Aktivität gegenüber bestimmten Mikroorganismen (Keimen) sowie ihrer Stabilität andererseits besteht. Des Weiteren gibt es keinen vorhersehbaren Zusammenhang zwischen der antimikrobiellen Wirkung, der toxikologischen Unbedenklichkeit, der Hautverträglichkeit und der Stabilität einer Substanz.

Gemäß einem ersten Aspekt betrifft die Erfindung Mischungen aus zwei, drei oder mehr unverzweigten 1,2-Alkandiolen, deren Kettenlängen (i) unterschiedlich sind und (ii) jeweils im Bereich von 5 bis 10 C-Atomen liegen. Hierbei sind die Anteile der besagten Diole in der Mischung vorzugsweise so eingestellt, dass ihre antimikrobielle Wirkung synergistisch verstärkt ist. Zu den besagten 1,2-Alkandiolen gehören insbesondere 1,2-Pentandiol, 1,2-Hexandiol, 1,2-Octandiol und 1,2-Decandiol. Neben den genannten 1,2-Alkandiol-Mischungen können weitere (z.B. übliche) antimikrobielle Wirkstoffe vorhanden sein, die zum Teil - wie unten weiter ausgeführt - eine weitere synergistische Wirkung entfalten können.

Die Erfindung beruht auf der überraschenden Erkenntnis, dass unverzweigte 1,2-Alkandiole mit einer Kettenlänge im Bereich von 5 bis 10 C-Atomen einen synergistisch verstärkten antimikrobiellen Effekt zumindest gegenüber ausgewählten Keimen zeigen, wenn man sie mit einem zweiten oder weiteren unverzweigten 1,2-Alkandiolen desselben Kettenlängenbereichs kombiniert, wobei die Kettenlängen des ersten und des zweiten bzw. der weiteren unverzweigten 1,2-Alkandiole allerdings unterschiedlich sind.

Insbesondere hat sich gezeigt, dass die erfindungsgemäßen Mischungen aus zwei, drei oder mehr unverzweigten 1,2-Alkandiolen unterschiedlicher Kettenlängen hervorragend zur Konservierung ansonsten verderblicher Artikel (siehe oben) verwendet werden können.

Möchte der Fachmann zur antimikrobiellen Behandlung einer Oberfläche (z.B. eines menschlichen oder tierischen Körpers) oder zur Konservierung eines ansonsten verderblichen Artikels (z.B. einer kosmetischen oder pharmazeutischen Formulierung) eine Mischung aus beispielsweise zwei unverzweigten 1,2-Alkandiolen einsetzen, deren Kettenlängen (i) unterschiedlich sind und (ii) jeweils im Bereich von 5 bis 10 C-Atomen liegen, kann er unter sämtlichen denkbaren Paarungen eine besonders geeignete Paarung auf folgendem Wege auswählen:
1. Bestimmen des unverzweigten 1,2-Alkandiols mit einer Kettenlänge im Bereich von 5 bis 10 C-Atomen mit der stärksten Einzelwirkung.
2. Kombinieren des 1,2-Alkandiols mit der stärksten Einzelwirkung mit jedem anderen unverzweigten 1,2-Alkandiol einer Kettenlänge im Bereich von 5 bis 10 C-Atomen.
3. Bestimmen derjenigen Kombination gemäß 2., welche die stärkste antimikrobielle Wirkung besitzt.

Mit der vorstehend skizzierten Vorgehensweise wird der Fachmann in der Regel zu der wirksamsten, immer aber zu einer sehr guten Kombination von 1,2-Alkandiolen im genannten Kettenlängenbereich gelangen. Er sei darauf hingewiesen, dass die erfindungsgemäßen Alkandiof-Mischungen zum Teil zwar gegenüber bestimmten Keimen eine synergistisch erhöhte antimikrobielle Wirkung besitzen, gegenüber anderen Keimen jedoch lediglich eine Wirkung, die der Summe ihrer Einzelwirkungen entspricht oder gar antagonistisch verschlechtert ist.

Obwohl sich die Fachwelt bereits umfangreich mit den antimikrobiellen Eigenschaften von 1,2-Diolen befasst hatte, gab es bislang keinen Hinweis, dass Mischungen aus zwei, drei oder mehr unverzweigten 1,2-Alkandiolen, deren Kettenlängen (i) unterschiedlich sind und (ii) jeweils im Bereich von 5 bis 10 C-Atomen liegen, eine im Einzelfall deuüich verbesserte antimikrobielle Wirkung (zumindest gegenüber ausgewählten Keimen) besitzen. Der Stand der Technik gab auch keine Anregung, derartige Mischungen (Kombinationen) als antimikrobielle Wirkstoffe einzusetzen.

So ist aus der JP 11322591 A zwar bekannt, dass die Dosis bestimmter konventioneller antiseptischer Mikrobizide reduziert werden kann, indem ein 1,2-Alkandiol mit beispielweise 5, 6 oder 8 C-Atomen in der Kette zugesetzt wird, doch ergibt sich hieraus nicht, dass eine Mischung aus zwei, drei oder mehr unverzweigten 1,2-Alkandiolen, deren Kettenlängen (i) unterschiedlich sind und (ii) jeweils im Bereich von 5 bis 10 C-Atomen liegen, einen antimikrobiellen (Kombinations-)Wirkstoff darstellen, welcher gegenüber zumindest ausgewählten Keimen eine extrem hohe Wirksamkeit besitzt.

Ganz ähnlich offenbart die JP 11310506 einen synergistischen Effekt bei der Kombination von Paraben mit 1,2-Pentandiol, 1,2-Hexandiol oder 1,2-Octandiol. Die der vorliegenden Erfindung zugrundliegende Erkenntnis der synergistischen Verstärkung der antimikrobiellen Eigenschaften der besagten 1,2-Alkandiole ergibt sich aus der JP 11310506 A jedoch ebenfalls nicht.

In der US 6,123,953 werden bestimmte synergistische Effekte zwischen einem 1,2-Alkandiol einer Kettenlänge im Bereich von 5 bis 14 Kohlenstoffatomen und einem Glyceryl-Polymethacrylat-Gel beschrieben. Das US-Dokument offenbart jedoch keine Kombination von 1,2-Alkandiolen und schon gar nicht derartige Kombinationen (Mischungen) mit einer antimikrobiellen Wirkung, welche gegenüber der Wirkung der Einzelsubstanzen erhöht ist.

Die EP 0 524 548 A1 offenbart bestimmte antimikrobiell wirksame Mischungen, die neben (A) einem antimikrobiell wirksamen aromatischen Alkohol der Formel 1, 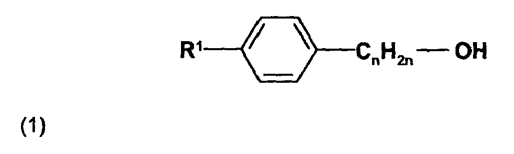 in der R² Wasserstoff oder eine Alkylgruppe mit 1 bis 4 C-Atomen und n eine ganze Zahl von 1 bis 6 ist, (B) ein antimikrobiell wirksames 1,2- oder 1,3-Diol der Formel R² -CHOH-(CHR³)ₓ-CH₂OH enthalten, wobei x = 0 oder 1 ist und wenn x = 0 ist, R² eine Alkylgruppe mit 6 bis 22 C-Atomen oder eine Alkoxymethyl- oder 2-Hydroxyalkoxymethylgruppe mit jeweils 6 bis 22 C-Atomen in der Alkoxygruppe ist, und wenn x = 1 ist, die Gruppe R² Wasserstoff ist und R³ eine der vorgenannten Bedeutungen von R² hat, wobei die Komponenten im Gewichtsverhältnis 9:1 bis 1:9 vorliegen. Die offenbarten antimikrobiell wirksamen Mischungen werden als geeignet zur Herstellung antiseptisch wirksamer Hautreinigungsmittel und zur Konservierung wässriger Zubereitungen mikrobiell abbaubarer oder verderblicher Stoffe bezeichnet. Diese technische Lehre legt es aber nicht nahe, zwei, drei oder mehr unverzweigte 1,2-Alkandiole, deren Kettenlängen (i) unterschiedlich sind und (ii) jeweils im Bereich von 5 bis 10 C-Atomen liegen, zu vermischen und die erhaltene Mischung als antimikrobiellen Wirkstoff einzusetzen. Insbesondere gibt die EP 0 524 548 A1 keinen Hinweis auf die Möglichkeit, dass derartige Alkandiol-Mischungen eine synergistisch verstärkte antimikrobielle (Kombinations-)Wirkung besitzen könnten, so dass insbesondere (wie im Rahmen der vorliegenden Erfindung möglich) auf die aromatischen Alkohole der Formel 1 verzichtet werden könnte.

Die JP 10053510 offenbart Kombinationen von Pentandiol und Phenoxyethanol als antiseptische Mittel für Kosmetika.

Die DE 199 24 496 A1 offenbart zur Bekämpfung menschlichen Körpergeruches Mischungen aus mindestens einem mehrwertigen Alkohol mit 5 bis 15 Kohlenstoffatomen (z.B. 1,2-Pentan-, Hexan- oder Octandiol) und einem Zitronensäure-Trialkylester.

In der EP 1 000 542 A1 sind Kombinationen aus 2,4,6-Cycloheptatrien-l-on-2-hydroxy-4(1-methyl-ethyl) mit bestimmten Polyolen beschrieben. Als Polyol können dabei auch 1,2-Octandiol, 1,2-Pentandiol und/oder Octoxyglycerin eingesetzt werden. Hierin ist aber kein Hinweis zu sehen auf die Verwendung einer Mischung aus 1,2-Octandiol und 1,2- Pentan diol oder einer sonstigen Mischung aus zwei, drei oder mehr unverzweigten 1,2-Alkandiolen als antimikrobieller Wirkstoff. Die Funktion der in der EP 1 000 542 A1 offenbarten 1,2-Alkandiole bleibt vielmehr unklar, insbesondere entsteht aus Absatz [0048] der EP 1 000 542 A1 der Eindruck, sie würden ohne Anwesenheit des besagten Cycloheptatrienon-Derivats (oder alternativ eines Natrium-Capryl-Lactyl-Lactylats) nur eine ungenügende antimikrobielle Wirksamkeit besitzen.

WO 99/56715 und WO 99/56716 befassen sich mit der Verwendung von 1,2-Hexandiol als Deodorant oder Antitranspirant. Auch sie offenbaren nicht eine synergistische Verstärkung der antimikrobiellen Wirksamkeit durch Kombinationen bestehend aus mindestens zwei Diolen der allgemeinen Formel 1.

Darüber hinaus befasst sich eine Reihe weiterer Dokumente mit Anwendungen von 1,2-Diolen, ohne jedoch auf eine synergistische Verstärkung von deren antimikrobieller Wirksamkeit durch Kombinationen bestehend aus mindestens zwei Diolen einzugehen. Hingewiesen sei insoweit auf die folgenden Dokumente: FR 2,755,371; WO 99/11237; WO 99/56715; JP 20 0044419; JP 11 335258; JP 10 053510; J. Food Sc. 42(3), 699-701; DE 199 24 496 und JP 20 0148720.

Angesichts der umfassenden Untersuchungen zur antimikrobiellen Wirksamkeit einzelner Diole mit einer Kettenlänge im Bereich von 5 bis 10 C-Atomen war es nun besonders überraschend, dass Mischungen aus zwei, drei oder mehr unverzweigten 1,2-Alkandiolen, deren Kettenlängen (i) unterschiedlich sind und (ii) jeweils im Bereich von 5 bis 10 C-Atomen liegen, eine stark synergistische Wirksamkeit zeigen und den einzeln dosierten 1,2-Diolen des gleichen Kettenlängenbereiches bei gleicher Konzentration insbesondere im Hinblick auf die Keimzeit-Reduktion deutlich überlegen sind. Insbesondere kann lediglich mit den besagten erfindungsgemäßen Mischungen im Einzelfall ein KBE-Wert (KBE = Anzahl koloniebildender Einheiten) von 0 erreicht werden.

Besonders bevorzugt ist die Verwendung einer Mischung aus (a) 1,2-Hexandiol und 1,2-Octandiol, (b) 1,2-Hexandiol und 1,2-Decandiol, (c) 1,2-Pentandiol, 1,2-Hexandiol und 1,2-Octandiol, (d) 1,2-Hexandiol, 1,2-Octandiol und 1,2-Decandiol oder (e) 1,2-Pentandiol, 1,2-Hexandiol und 1,2-Decandiol. Vergleiche hierzu auch die Beispiele weiter unten.

Bezogen auf die Gesamtmasse der Mischung der Diole sollte der Anteil jedes einzelnen Diols im Bereich von 1 bis 99 Gew.% liegen, vorzugsweise aber im Bereich von 20 bis 80 Gew.-%.

Die erfindungsgemäßen 1,2-Diol-Mischungen sind nicht nur zur Konservierung verderblicher Produkte wie z.B. Kosmetikprodukten, pharmazeutischen Produkten oder Lebensmitteln geeignet, sondern können aufgrund ihrer synergistisch verstärkten antimikrobiellen Wirksamkeit auch
(a) zur kosmetischen Behandlung von Körpergeruch verursachenden Mikroorganismen,
(b) zur kosmetischen Behandlung von Akne verursachenden Mikroorganismen,
(c) zur kosmetischen Behandlung von Mykosen verursachenden Mikroorganismen und
(d) zur Behandlung von Mikroorganismen auf oder in unbeliebter Materie
eingesetzt werden. Die erfindungsgemäßen Mischungen entfalten ihre synergistische Wirkung gegenüber einer Vielzahl von gram-positiven Bakterien, gram-negativen Bakterien, Schimmelpilzen und Hefen. Eine besonders gute Wirkung besteht gegenüber gram-negativen Bakterien wie *Escherichia coli* und *Pseudomonas aeruginosa,* gegenüber Hefen wie *Candida albicans* und gegenüber Pilzen wie *Aspergillus niger.* Als besonders vorteilhaft ist hierbei die sehr gute Wirksamkeit der erfindungsgemäßen 1,2-Diol-Mischungen gegenüber *Aspergillus niger,* einem nur sehr schwer zu bekämpfenden Schimmelpilz, zu betrachten, denn bei Verwendung einzelner 1,2-Diote mit einer Kettenlänge im Bereich von 5 bis 10 C-Atomen lässt sich dessen KBE-Wert nach eigenen Untersuchungen nicht bis auf den Wert 0 reduzieren.

Die vorliegende Erfindung betrifft auch entsprechende Verfahren zur kosmetischen und/oder therapeutischen Behandlung von Keimen, und zwar insbesondere von (a) Körpergeruch verursachenden Mikroorganismen, (b) Akne verursachenden Mikroorganismen und/oder (c) Mykosen verursachenden Mikroorganismen, umfassend die topische Applikation einer antimikrobiell wirksamen Menge eines Gemisches aus zwei, drei oder mehr unverzweigten 1,2-Alkandiolen, deren Kettenlängen (i) unterschiedlich sind und (ii) jeweils im Bereich von 5 bis 10 C-Atomen liegen, wobei die Anteile der besagten Diole in der Mischung so eingestellt sind, dass ihre antimikrobielle Wirkung synergistisch verstärkt ist.

Bevorzugte Ausgestaltungen des erfindungsgemäßen Verfahrens entsprechen den vorstehend erläuterten bevorzugten Ausgestaltungen der erfindungsgemäßen Verwendung.

Die menschliche Haut wird von einer Vielzahl verschiedener Mikroorganismen besiedelt, zu denen die vorstehend bereits genannten Mikroorganismen ebenso wie andere gehören. Die meisten dieser Mikroorganismen sind nicht pathogen und für den physiologischen Zustand der Haut und für deren Geruch irrelevant. Andere hingegen können den gesunden Zustand der Haut maßgeblich beeinflussen. Einige, die menschliche Hautflora stark beeinflussende Mikroorganismen sind in Tabelle 1 dargestellt.

Wie eigene Untersuchungen jetzt zeigten, sind die erfindungsgemäßen synergistisch wirksamen Mischungen aus zwei, drei oder mehr unverzweigten 1,2-Alkandiolen, deren Kettenlängen (i) unterschiedlich sind und (ii) jeweils im Bereich von 5 bis 10 C-Atomen liegen, nicht nur gegenüber den vorstehend bereits bezeichneten Keimen sondern auch gegenüber *Staphylococcus epidermidis, Brevibactsrium epidermidis, Propionibacterium acnes* sowie gegenüber *Trichophyton*- und *Epidermophyton*-Arten gut wirksam, so dass sie auch als Mittel zur Behandlung (Bekämpfung) von Achsel- und Fußgeruch bzw. Körpergeruch im allgemeinen, als Mittel zur Bekämpfung von Akne, als Antischuppenmittel und zur Behandlung von Mykosen (insbesondere Dermatonmykosen) eingesetzt werden können (vergleiche erneut Tabelle 1).

**Tabelle 1:**

| **Mikroorganismen:** | |
|---|---|
| | |
| *Staphylococcus epidermidis* | Achselgeruch; allg. Körpergeruch |
| *Staphylococcus aureus* | Atopische Ekzeme; Wundinfektion |
| *Corynebacferium xerosis* | Achseigeruch |
| *Brevibacterium epidermidis* | Achselgeruch; Fuageruch |
| *Propionibacterium acnes* | Akne |
| *Escherichia coli* | Wundinfektionen |
| *Pseudomonas aeruginosa* | Wundinfektionen |
| *Malassezia furfur (syn. Pityrosporum* ovale) | Schuppenbildung |
| *Candida albicans* | Allg. Candidosen |
| *Trichophyton mentagrophytes* | Haut- und Nagelmykosen |
| *Trichophyton rubrum* | Haut- und Nagelmykosen |
| *Epidermophyton floccosum* | Haut- und Nagelmykosen |
| *Aspergillus niger* | Schimmelbefall |

Hierzu sei ergänzend folgendes bemerkt:

Durch den bakteriellen Abbau im Schweiß enthaltener, körpereigener Stoffe wie z.B. ungesättigter Fettsäuren entstehen aus mehr oder minder schwach riechenden Vorstufen unangenehm riechende Zersetzungsprodukte, die das körperüche Wohlbefinden stark beeinflussen können. Zur Verhinderung der Entstehung der für Körpergeruch verantwordichen Substanzen verwendet man in der Kosmetik Produkte, die entweder die Bildung von Körperschweiß unterbinden (sogenannte Antiperspirantien) oder Substanzen, die das Wachstum der für die Geruchsbildung verantwortlichen Bakterien der menschlichen Haut hemmen (Deodorantien). Bakterienarten wie *Staphylococcus epidermidis, Corynebacterium xerosis* und *Brevibacterium epidermidis* sind maßgeblich für die Bildung von Achsel- und Fußgeruch, bzw. Körpergeruch im allgemeinen verantwortlich. In der kosmetischen Industrie besteht daher ein beständiger Bedarf an neuen Mitteln zur Behandlung dieser und anderer Körpergeruch (einschließlich Achsel- und Fußgeruch) verursachenden Mikroorganismen.

Ein Akne verursachender Mikroorganismus ist *Propionibacterium acnes*, bei dem es sich um einen anaerob wachsenden Keim handelt. Die kosmetische Industrie sucht beständig nach Mitteln zur Behandlung dieses Keimes und anderer Akne verursachender Mikroorganismen.

Alle Bereiche der menschlichen Haut können von Mykosen (insbesondere Dermatomykosen und Nagelmykosen) befatlen werden. Besonders häufig sind Hautateale betroffen, auf weichen sich durch das Tragen von Kleidung, Schuhwerk oder Schmuck Feuchtigkeit und Wärme stauen können. Als besonders unangenehm empfunden werden Pilzerkrankungen der Finger- und Fußnägelbereiche. Maßgeblich verantwortlich für die Bildung von Mykosen sind häufig verschiedene *Trichophyton*- und *Epidermophyton*-Arten. Die kosmetische Industrie sucht beständig nach neuen Mitteln zur Behandlung dieser und anderer Mykosen verursachenden Mikroorganismen.

Unter "Behandlung" wird dabei im Rahmen des vorliegenden Textes jede Form der Einflussnahme auf die betreffenden Mikroorganismen verstanden, bei der die Vermehrung dieser Mikroorganismen gehemmt und/oder die Mikroorganismen getötet werden.

Vorzugsweise liegt die Einsatzkonzentration der erfindungsgemäßen Mischungen aus zwei, drei oder mehr unverzweigten 1,2-Alkandiolen, deren Kettenlängen (i) unterschiedlich sind und (ii) jeweils im Bereich von 5 bis 10 C-Atomen, bei Verwendung als Konservierungsmittel in einem Nahrungsmittel oder als antimikrobieller Wirkstoff in einem kosmetischen bzw. pharmazeutischen Endprodukt im Bereich zwischen 0,01 und 30 Gew.%, besonders bevorzugt aber im Bereich zwischen 0,1 und 5 Gew.%, jeweils bezogen auf die Gesamtmasse des Nahrungsmittels bzw. Endprodukts.

Auch in einem bevorzugten erfindungsgemäßen Verfahren zur kosmetischen und/oder therapeutischen Behandlung von (a) Körpergeruch verursachenden Mikroorganismen, (b) Akne verursachenden Mikroorganismen und/oder (c) Mykosen verursachenden Mikroorganismen liegt die Einsatzkonzentration der erfindungsgemäßen, synergistisch wirksamen Mischungen im Bereich zwischen 0,01 und 30 Gew.-% und besonders bevorzugt im Bereich zwischen 0,1 und 5 Gew.%, jeweils bezogen auf die Gesamtmasse des kosmetischen oder pharmazeutischen Produktes, welches die Mischung umfasst.

Die synergistisch wirksamen Diolgemische können hierbei (a) prophylaktisch oder (b) im Bedarfsfall zum Einsatz kommen.

Die Konzentration der z.B. täglich zu applizierenden Wirkstoffmenge ist unterschiedlich und hängt vom physiologischen Zustand des Probanden sowie individualspezifischen Parametern wie Alter oder Körpergewicht ab. Die erfindungsgemäßen synergistisch wirksamen Diolgemische können sowohl allein, als auch in Kombination mit weiteren antimikrobiell wirksamen Substanzen zum Einsatz gelangen.

Es sei darauf hingewiesen, dass der Begriff 1,2-Diol im Rahmen des vorliegenden Textes sowohl das entsprechende 2S-konfigurierte Enantiomer als auch das 2R-konfigurierte Enantiomer sowie beliebige Mischungen aus diesen 2S- und 2R-konfigurierten Enantionmeren umfasst. Aus kommerziellen Gründen ist es zwar besonders vorteilhaft, Gemische von Razematen der jeweiligen Diole zur Bekämpfung von Mikroorganismen einzusetzen, da diese synthetisch besonders leicht zugänglich sind, die reinen Enantiomere oder nicht-razemische Mischungen dieser Enantiomere sind aber ebenfalls für die erfindungsgemäßen Zwecke geeignet.

Die vorliegende Erfindung betrifft schließlich auch entsprechende antimikrobielle Zusammensetzungen, umfassend:
(a) eine Mischung aus zwei, drei oder mehr unverzweigten 1,2-Alkandiolen, deren Kettenlängen (i) unterschiedlich sind und (ii) jeweils im Bereich von 5 bis 10 C-Atomen liegen, und
(b) eine zu der besagten Mischung kompatible Trägersubstanz. Sowie Antimikrobielle Zusammensetzung, umfassend:
(c) als antimikrobiellen Wirkstoff eine Mischung aus zwei, drei oder mehr unverzweigten 1,2-Alkandiolen, deren Kettenlängen (i) unterschiedlich sind und (ii) jeweils im Bereich von 5 bis 10 C-Atomen liegen,
   und
(a) eine zu der besagten Mischung kompatible Trägersubstanz sowie gegebenenfalls
(b) einen weiteren antimikrobiellen Wirkstoff, der kein unverzweigtes 1,2-Alkandiol umfasst.

Hinsichtlich der bevorzugten Ausgestaltungen der erfindungsgemäßen antimikrobiellen Zusammensetzungen gilt das Vorgesagte entsprechend.

Weitere erfindungsgemäße Verwendungen/Verfahren und Zusammensetzungen lassen sich den nachfolgenden Ausführungen und den beigefügten Patentansprüchen entnehmen.

Zusammensetzungen, die ein synergistisch wirksames erfindungsgemäßes Gemisch enthalten, werden, insbesondere soweit sie gegen Körpergeruch verursachende Keime eingesetzt werden, in der Regel topisch in Form von Lösungen, Cremes, Lotionen, Gelen, Sprays oder dergleichen appliziert. Für andere Zwecke ist in manchen Fällen eine orale (Tabletten, Kapseln, Pulver, Tropfen), intravenöse, intraokulare, intraperitoneale oder intramuskuläre Applikation oder eine Applikation in Form eines imprägnierten Verbands sinnvoll.

Die erfindungsgemäß verwendeten synergistisch wirksamen Mischungen von 1,2-AJkandiolen lassen sich ohne Schwierigkeiten in gängige kosmetische oder dermatologische Formulierungen wie u.a. Pumpsprays, Aerosolsprays, Cremes, Salben, Tinkturen, Lotionen, Nagelpflegeprodukte (z.B. Nagellacke, Nagellackentferner, Nagelbalsame) und dergleichen einarbeiten. Hierbei ist es auch möglich und in manchen Fällen vorteilhaft, die erfindungsgemäß verwendeten synergistischen Mischungen von 1,2-Alkandiolen mit weiteren Wirkstoffen zu kombinieren, beispielsweise mit anderen antimikrobiell, antimykotisch bzw. antiviral wirksamen Stoffen. Die synergistisch wirksame 1,2-Alkandiole enthaltenden kosmetischen und/oder dermatologischen/keratologischen Formulierungen können hierbei ansonsten wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Werden die erfindungsgemäßen synergistischen Mischungen von 1,2-Alkandiolen als Wirkstoffe zur Konservierung organischen Materials eingesetzt, so können vorteilhaft zusätzlich ein weiteres oder mehrere weitere Konservierungsmittel eingesetzt werden. Vorzugsweise gewählt werden hierbei Konservierungsmittel wie Benzoesäure, ihre Ester und Salze, Propionsäure und ihre Salze, Salicylsäure und ihre Salze, 2,4-Hexadiensäure (Sorbinsäure) und ihre Salze, Formaldehyd und Paraformaldehyd, 2-Hydroxybiphenylether und seine Salze, 2-Zinksulfidopyridin-N-oxid, anorganische Sulfite und Bisulfite, Natriumjodat, Chlorbutanolum, 4-Ethylquecksilber-(II)5-Amino-1,3-bis(2-Hydroxybenzoesäure, ihre Salze und Ester, Dehydratcetsäure, Ameisensäure, 1,6-Bis(4-amidino-2-bromphenoxy)-n-hexan und seine Salze, das Natriumsalz der Ethylquecksilber-(II)-thiosalicylsäure, Phenylquecksilber und seine Salze, 10-Undecylensäure und ihre Salze, 5-Amino-1,3-bis(2-ethylhexyl)-5-methylhexahydropyrimidin, 5-Brom-5-nitro-1,3-dioxan, 2-Brom-2-nitro-1,3-propandiol, 2,4-Dichlorbenzylalkohol, N-(4-Chlorphenyl)-N'-(3,4-dichlorphenyl)-hamstoff, 4-Chlor-m-kresol, 2,4,4'-Trichlor-2'-hydroxy-diphenylether, 4-Chlor-3,5-dimethylphenol, 1,1'-Methylen-bis(3-(1-hydroxymethy)-2,4-dioximidazolidin-5-yl)harnstoff), Poly-(hexamethylendiguanid)-hydrochlorid, 2-Phenoxyethanol, Hexamethylentetramin, 1-(3-Chloroallyl)-3,5,7-triaza-1-azonia-adamantanchlorid, 1(4-Chlorphenoxy)1(1H-imidazol-1-yl)-3,3-dimethyl-2-buta-non, 1,3-Bis-(hydroxymethyl)-5,5-dimethyl-2,4-imidazolidindion, Benzylalkohol, Octopirox, 1,2-Dibrom-2,4-dicyanobutan, 2,2'-Methylen-bis(6-brom-4-chlor-phenol), Bromchlorophen, Mischung von 5-Chlor-2-methyl-3(2H)-isothiazolinon und 2-Methyl-3(2H)isothiazlinon mit Magnesiumchlorid und Magnesiumnitrat, 2-Benzyl-4-chlorphenol, 2-Chloracetamid, Chlorhexidin, Chlorhexidinacetat, Chlorhexidingluconat, Chlorhexidinhydrochlorid, 1-Phenoxy-propan-2-ol, N-Alkyl(C₁₂-C₂₂)trimethyl-ammoniumbromid und -chlorid, 4,4-Dimethyl-1,3-oxazolidin, N-Hydroxymethyl-N-(1,3-di(hydroxymethyl)-2,5-dioxoimidazolidin-4-yl)-N'-hydroxy-methylhamstoff, 1,6-Bis(4-amidino-phenoxy)-n-hexan und seine Salze, Glutaraldehyd, 5-Ethyl-1-aza-3,7-dioxabicyclo(3.3.0)octan, 3-(4-Chlorphenoxy)-1,2-propandiol, Hyamine, Alkyl-(C₈-C₁₈)-dimethyl-benzyl-ammoniumchlorid, Alkyl-(C₈-C₁₈)-dimethyl-benzylammonium-bromid, Alkyl-(C₈-C₁₈)-dimethyl-benzylammoniumsaccharinat, Benzylhemiformal, 3-Jod-2-propinyl-butylcarbamat, Natrium-hydroxymethyl-aminoacetat oder Natrium-hydroxymethyl-aminoacetat.

Der überraschend zu erzielende Vorteil von Wirkstoffkombinationen bestehend aus (a) einer erfindungsgemäßen synergistischen Mischung von 1,2-Alkandiolen und (b) mindestens einem weiteren Konservierungsmittel wird anhand von Beispiel 3 näher erläutert werden.

Sollen die erfindungsgemäßen synergistischen Mischungen von 1,2-Alkandiolen vornehmlich zur Inhibition des Wachstums unerwünschter Mikroorganismen auf oder in tierischen Organismen eingesetzt werden, so ist eine Kombination mit weiteren antibakteriellen oder antimykotischen Wirkstoffen auch hier in manchen Fällen vorteilhaft. Erwähnenswert sind insoweit als weitere Wirkstoffe neben der großen Gruppe der klassischen Antibiotika insbesondere die für Kosmetika relevanten Produkte wie Triclosan, Climbazol, Octoxyglycerin , Octopirox (1-Hydroxy-4-methyl-6-(2,4,4-trimethylpentyl)-2(1H)-pyridone, 2-aminoethanol), Chitosan, Farnesol, Glycerinmonolaurat oder Kombinationen der genannten Substanzen, die u.a. gegen Achselgeruch, Fußgeruch oder Schuppenbildung eingesetzt werden.

Darüber hinaus können die synergistischen Mischungen von 1,2-Alkandiolen auch in Kombination mit schweißhemmenden Wirkstoffen (Antitranspirantien) besonders vorteilhaft zur Bekämpfung von Körpergeruch eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Für die Anwendung in kosmetischen und dermatologischen Antitranspirantien haben sich im wesentlichen die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride. Neben Aluminiumsalzen kommen auch weitere Substanzen in Betracht wie zum Beispiel a) eiweißfällende Substanzen wie u.a. Formaldehyd, Glutaraldehyd, natürliche und synthetische Gerbstoffe sowie Trichloressigsäure, die einen oberflächlichen Verschluß der Schweißdrüsen herbeiführen b) Lokalanästhetika (u.a. verdünnte Lösungen von z.B. Lidokain, Prilokain oder Gemischen derartiger Substanzen), die durch Blockade der peripheren Nervenbahnen die sympathische Versorgung der Schweißdrüsen ausschalten, c) Zeolithe vom X-, A- oder Y-Typ, die neben der Reduktion der Schweißsekretion auch als Adsorbentien für schlechte Gerüche fungieren und d) Botulinustoxin (Toxin des Bakteriums *Chlostridium botulinum*), welches auch bei Hyperhidrose, einer krankhaft erhöhten Schweißsekretion, zum Einsatz gelangt und dessen Wirkung auf einer irreversiblen Blockierung der Freisetzung der für die Schweißsekretion relevanten Transmittersubstanz Acetylcholin beruht.

Sollen die erfindungsgemäßen, synergistischen Mischungen von 1,2-Alkandiolen zur antimikrobiellen Behandlung einer Oberfläche (z.B. eines menschlichen oder tierischen Körpers) eingesetzt werden, so ist in manchen Fällen eine Kombination mit (Metall)-Chelatoren vorteilhaft. Bevorzugt einzusetzende (Metall)-Chelatoren sind hierbei u.a α-Hydroxyfettsäuren, Phytinsäure, Lactoferrin, α-Hyroxysäuren wie u.a. Zitronensäure, Milchsäure und Äpfelsäure sowie Huminsäuren, Gallensäuren, Gallenextrakte, Bilirubin, Biliverdin bzw. EDTA, EGTA und deren Derivate.

Zur Anwendung werden die erfindungsgemäßen kosmetischen und/oder dermatologisch wirksamen synergistischen Mischungen von 1,2-Atkandiolen in der für Kosmetika und Dermatika üblichen Weise auf die Haut und/oder die Haare in ausreichender Menge aufgebracht. Besondere Vorteile bieten dabei kosmetische und dermatologische Zubereitungen, die eine erfindungsgemäße Mischung enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Ö! (W/O) oder vom Typ Ölin-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Wie erwähnt können Zubereitungen, die eine synergistische Mischung von 1,2-Alkandiolen enthalten, vorteilhaft mit Substanzen kombiniert werden, die UV-Strahlung absorbieren, wobei die Gesamtmenge der Filtersubstanzen z.B. 0,01 Gew.-% bis 40 Gew.-%, vorzugsweise 0,1% bis 10 Gew.-%, insbesondere 1,0 bis 5,0 Gew.% beträgt, bezogen auf das Gesamtgewicht der Zubereitungen, um kosmetische Zubereitungen zur Verfügung zu stellen, die das Haar bzw. die Haut vor ultravioletter Strahlung schützen.

Enthalten die erfindungsgemäßen Zubereitungen UVB-Filtersubstanzen, können diese öllöslich oder wasserlöslich sein. Vorteilhafte öllösliche UVB-Filter sind z.B.: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoesäure(2-ethylhexyl)ester, 4-(Dimethyl-amino) benzoe-säureamylester; Ester der Zimtsäure, vorzugsweise 4-Methoxyzimtsäure(2-ethylhexyl)ester, 4-Methoxyzimtsäureisopentylester; Ester der Salicylsäure, vorzugsweise Salicylsäure(2-ethylhexyl)ester, Salicylsäure (4isopropylbenzyl) ester, Salicylsäurehomomenthylester, Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzaimalonsäuredi(2-ethylhexyl) ester, 2,4,6-Trianilino-(p-carbo-2'-ethyl-1'-hexyloxy)-1,3,5-triazin. Vorteilhafte wasserlösliche UVB-Filter sind z.B. Salze der 2-Phenylbenzimidazol-5-sulfonsäure wie ihr Natrium-, Kalium- oder ihr Triethanolammonium-Salz, sowie die Sulfonsäure selbst; Sulfonsäure-Derivate von Benzophenonen, vorzugsweise 2-Hydroxy-4-methoxybenzophenon-5-sulfonsäure und ihre Salze; Sulfonsäure-Derivate des 3-Benzylidencamphers, wie z.B. 4-(2-Oxo-3-bornylidenmethyl) benzolsulfonsäure, 2-Methyl-5-(2-oxo-3-bomylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet.

Die vorstehende Liste der genannten UVB-Filter, die in Kombination mit den erfindungsgemäßen synergistischen Mischungen von 1,2-Alkandiolen verwendet werden können, soll selbstverständlich nicht als abschließend verstanden werden. Es kann auch von Vorteil sein, UVA-Filter einzusetzen, wie sie üblicherweise in kosmetischen Zubereitungen enthalten sind. Bei diesen Substanzen handelt es sich vorzugsweise um Derivate des Dibenzoylmethans, insbesondere um 1-(4'-tert.-Butylphenyl)-3-(4'-methoxyphenyl) propan-1,3-dion und um 1-Phenyl-3-(4'-isopropylphenyl)propan-1,3-dion.

Die erfindungsgemäßen, synergistischen Mischungen von 1,2-Alkandiolen können in kosmetischen Zubereitungen vorteilhaft mit kosmetischen Hilfsstoffen kombiniert werden, wie sie üblicherweise in solchen Zubereitungen verwendet werden, also z.B. mit: Antioxidantien; Parfümölen; Mitteln zum Verhindern des Schäumens; Farbstoffen; Pigmente, die eine färbende Wirkung haben; Verdickungsmittel; obetHächenaktive Substanzen; Emulgatoren; weichmachende Substanzen; anfeuchtende und/oder feuchthaltende Substanzen; Fette; Öle; Wachse; andere übliche Bestandteile einer kosmetischen Formulierung wie Alkohole, Polyole, Polymere, Schaumstabilisatoren, Elektrolyte, organische Lösungsmittel oder Silikonderivate.

In synergistisch wirksame Mischungen von 1,2-Alkandiolen enthaltenden Formulierungen zur topischen prophylaktischen oder kosmetischen Behandlung der Haut ist regelmäßig ein hoher Anteil an pflegenden Substanzen vorteilhaft. Gemäß einer bevorzugten Ausführungsform enthalten die Zusammensetzungen ein oder mehrere pflegende tierische und/oder pflanzliche Fette und Öle wie Olivenöl, Sonnenblumenöl, raffiniertes Sojaöl, Palmöl, Sesamöl, Rapsöl, Mandelöl, Borretschöl, Nachtkerzenöl, Kokosöl, Sheabutter, Jojobaöl, Spermöl, Rindertalg, Klauenöl und Schweineschmalz sowie gegebenenfalls weitere pflegende Bestandteile wie zum Beispiel Fettalkohole mit 8-30 C-Atomen. Die Fettalkohole können hierbei gesättigt oder ungesättigt bzw. linear oder verzweigt sein. Einsetzbar sind zum Beispiel Decanol, Decenol, Octanol, Octenol, Dodecanol, Dodecenol, Octadienol, Decadienol, Dodecadienoi, Oleylalkohol, Ricinolalkohol, Erucaalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei die Aufzählung durch weitere strukturchemisch verwandte Alkohole nahezu beliebig erweiterbar wäre. Die Fettalkohole stammen bevorzugt von natürlichen Fettsäuren ab, wobei sie üblicherweise aus den korrespondierenden Estern der Fettsäuren durch Reduktion hergestellt werden. Einsetzbar sind weiterhin Fettalkoholfraktionen, die durch Reduktion aus natürlich vorkommenden Fetten und fetten Ölen, wie z.B. Rindertalg, Erdnußöl, Rüböl, Baumwollsamenöl, Sojaöl, Sonnenblumenöl, Palmkemöl, Leinöl, Maisöl, Rizinusöl, Rapsöl, Sesamöl, Kakaobutter und Kokosfett entstehen.

Zu pflegenden Substanzen, die sich vorzüglich mit den erfindungsgemäßen synergistische Mischungen von 1,2-Alkandiolen kombinieren lassen, zählen darüber hinaus auch
- Ceramide, wobei man unter Ceramiden N-Acylsphingosine (Fettsäreamide des Sphingosins) oder synthetische Analoga solcher Lipide (sogenannte Pseudo-Ceramide) versteht, die das Wasserhaltevermögen des Stratum Corneums deutlich verbessern.
- Phospholipide, beispielsweise Sojalecithin, Ei-Lecithin und Kephaline
- Vaseline, Paraffin- und Silikonöle; zu letzteren zählen unter anderem Dialkyl- und Alkylarylsiloxane wie Dimethylpolysiloxan und Methylphenylpolysiloxan, sowie deren alkoxylierte und quaternierte Derivate.

Den erfindungsgemäßen synergistischen Mischungen von 1,2-Alkandiolen können vorteilhaft auch tierische und/oder pflanzliche Proteinhydrolysate zugesetzt werden. Vorteilhaft sind insoweit insbesondere Elastin-, Kollagen-, Keratin-, Milcheiweiß-, Sojaprotein-, Haferprotein-, Erbsenprotein-, Mandelprotein-, und Weizenproteinfraktionen oder entsprechende Proteinhydrolysate, aber auch deren Kondensationsprodukte mit Fettsäuren sowie quarternisierte Proteinhydrolysate, wobei die Verwendung pflanzlicher Proteinhydrolysate bevorzugt ist.

Sofem eine kosmetische oder dermatologische, eine erfindungsgemäße synergistische Mischung von 1,2-Alkandiolen enthaltende Zubereitung eine Lösung oder Lotion darstellt, können als Lösungsmittel verwendet werden:
- Wasser oder wäßrige Lösungen;
- fette Öle, Fette, Wachse und andere natürliche und synthetische Fettkörper, vorzugsweise Ester von Fettsäuren mit Alkoholen niedriger C-Zahl, z.B. mit lsopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte. Insbesondere werden Gemische der vorstehend genannten Lösungsmittel verwendet. Bei alkoholischen Lösungsmitteln kann Wasser ein weiterer Bestandteil sein.

Kosmetische Zubereitungen, die erfindungsgemäße, synergistische Mischungen von 1,2-Alkandiolen enthalten, können auch Antioxidantien enthalten, wobei alle für kosmetische und/oder dermatologische Anwendungen geeigneten oder gebräuchlichen Antioxidantien verwendet werden können. Vorteilhaft werden die Antioxidantien gewählt aus der Gruppe bestehend aus Aminosäuren (z.B. Glycin, Histidin, Tyrosin, Tryptophan) und deren Derivaten, Imidazolen (z.B. Urocaninsäure) und deren Derivaten, Peptiden wie D, L-Carnosin, D-Camosin, L-Carnosin und deren Derivaten (z.B. Anserin), Carotinoiden, Carotinen (z.B. α-Carotin, β-Carotin, Lycopin) und deren Derivaten, Liponsäure und deren Derivaten (z.B. Dihydroliponsäure), Aurothioglucose, Propylthiouracil und anderen Thiolen (z.B. Thioredoxin, Glutathion, Cystein, Cystin, Cystamin und deren Glycosyl-, N-Acetyl-, Methyl-, Ethyl-, Propyl-, Amyl-, Butyl- undLauryl-Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren, z.B. α-Hydroxyfettsäuren, Palmitinsäure, Phytinsäure, Lactoferrin, α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linotensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherote und deren Derivate (z.B. Vitamin-Vitamin-E-acetat), Vitamin A und dessen Derivate (Vitamin-A-palmitat) sowie Coniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, trans-Stilbenoxid) sowie Derivate (Salze, Ester, Ether, Zucker, Nukleotide, Nukleoside, Peptide und Lipide) dieser genannten Wirkstoffe.

Kosmetische Zubereitungen, die erfindungsgemäße, synergistische Mischungen von 1,2-Alkandiolen enthalten, können auch Vitamine und Vitaminvorstufen enthalten, wobei alle für kosmetische und/oder dermatologische Anwendungen geeigneten oder gebräuchlichen Vitamine und Vitaminvorstufen verwendet werden können. Erwähnenswert sind hier insbesondere Vitamine und Vitaminvorstufen wie Tocopherole, Vitamin A, Niacinsäure und Niacinsäureamid, weitere Vitamine des B-Komplexes, insbesondere Biotin und Vitamin C, Panthenol und dessen Derivate, insbesondere die Ester und Ether des Panthenols sowie kationisch derivatisierte Panthenole wie z.B. Panthenoltriacetat, Panthenolmonoethylether und dessen Monoacetat sowie kationische Parithenolderivate.

Kosmetische Zubereitungen, die erfindungsgemäße, synergistische Mischungen von 1,2-Alkandiolen enthalten, können auch entzündungshemmende bzw. rötungs- bzw. juckreizlindernde Wirkstoffe enthalten. Es können hierbei alle für kosmetische und/oder dermatologische Anwendungen geeigneten oder gebräuchlichen entzündungs-hemmenden bzw. rötungs- und juckreizlindernden Wirkstoffe verwendet werden. Vorteilhaft werden als entzündungshemmende bzw. rötungs- und juckreizlindernde Wirkstoffe steroidale entzündungshemmende Substanzen vom Kortikosteroiden-Typ eingesetzt wie z.B. Hydrocortison, Dexamethason, Dexamethasonphosphat, Methylprednisolon oder Cortison, wobei die Auflistung durch Zusatz weiterer steroidater Entzündungshemmer erweiterbar ist. Auch nichtsteroidale Entzündungshemmer können eingesetzt werden. Beispielhaft erwähnt werden sollen hier Oxicame wie Piroxicam oder Tenoxicam; Salicylate wie Aspirin, Disalcid, Solprin oder Fendosal; Essigsäre-Derivate wie Diclofenac, Fenclofenac, Indomethacin, Sulindac, Tolmetin, oder Clindanac; Fenamate wie Mefenamic, Medofenamic, Flufenamic oder Niflumic; Propionsäure-Derivate wie lbuprofen, Naproxen, Benoxaprofen oder Pyrazole wie Phenylbutazon, Oxyphenylbutazon, Febrazon oder Azapropazon. Alternativ können natürliche entzündungshermmende bzw. rötungs- und juckreizlindernde Stoffe eingesetzt werden. Einsetzbar sind Pflanzenextrakte, spezielle hochwirksame Pflanzenextrakt-Fraktionen sowie aus Pflanzenextrakten isolierte hochreine Wirksubstanzen. Besonders bevorzugt sind Extrakte, Fraktionen und Wirksubstanzen aus Kamille, Aloe vera, Commiphora-Arten, Rubia-Arten, Weiden, Weidenröschen, Hafer sowie Reinsubstanzen wie u.a. Bisabolot, Apigenin-7-glucosid, Boswelliasäure, Phytosterole, Glycyrrhizin-säure, Glabridin oder Licochalkon A. Die synergistische Mischungen von 1,2-Alkandiolen enthaltenden Formulierungen können auch Gemische aus zwei oder mehreren antünflammatorischen Wirkstoffen enthalten.

Kosmetische Zubereitungen, die erfindungsgemäße, synergistische Mischungen von 1,2-Alkandiolen enthalten, können auch Wirkstoffe mit hautaufhellender Wirkung enthalten. Erfindungsgemäß können hierbei alle für kosmetische und/oder dermatologische Anwendungen geeigneten oder gebräuchlichen hautaufhellenden Wirkstoffe verwendet werden. Vorteilhafte hautaufhellende Wirkstoffe sind insoweit Kojicacid, Hydrochinon, Arbutin, Ascorbinsäure, Magnesiumascorbylphosphat, Süßholzwurzelextrakten sowie deren Bestandteile Glabridin oder Licochalkon A, Extrakten von Rumex- und Ramulus-Arten, Extrakte aus Kieferarten (Pinus) und Extrakte aus Vitis- Arten, die u.a. hautaufhellende Stilbenderivate enthalten.

Kosmetische Zubereitungen, die erfindungsgemäße, synergistische Mischungen von 1,2-Alkandiolen enthalten, können auch Wirkstoffe mit hautbräunender Wirkung enthalten. Es können insoweit alle für kosmetische und/oder dermatologische Anwendungen geeigneten oder gebräuchlichen hautbräunenden Wirkstoffe verwendet werden. Beispielhaft sei hier das Dihydroxyaceton (DHA; 1,3-Dihydroxy-2-propanon) erwähnt. DHA kann sowohl in monomerer als auch in dimerer Form vorliegen, wobei in kristalliner Form der Anteil an Dimeren überwiegt.

Kosmetische Zubereitungen, die erfindungsgemäße, synergistische Mischungen von 1,2-Alkandiolen enthalten, können auch Mono-, Di- und Oligosaccharide wie beispielsweise Glucose, Galactose, Fructose, Mannose, Fruchtzucker und Lactose enthalten.

Kosmetische Zubereitungen, die erfindungsgemäße, synergistische Mischungen von 1,2-Alkandiolen enthalten, können auch Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden. Hinsichtlich der verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt beginnenden Tabelle aufgeführt sind. Vorteilhaft sind insbesondere die Extrakte aus Aloe, Hamamelis, Algen, Eichenrinde, Weidenröschen, Brennnessel, Taubnessel, Hopfen, Kamile, Schafgarbe, Arnika, Calendula, Klettenwurzel, Schachtelhalm, Weißdorn, Lindenblüten, Mandel, Fichtennadel, Roßkastanie, Sandelholz, Wacholder, Kokosnuß, Mango, Aprikose, Orange, Zitrone, Limette, Grapefruit, Apfel, grünem Tee, Grapefruitsamen, Weizen, Hafer, Gerste, Salbei, Thymian, Quendel, Rosmarin, Birke, Malve, Wiesenschaumkraut, Weidenrinde, Hauhechel, Huflattich, Eibisch, Ginseng und Ingerwurzel. Besonders bevorzugt sind dabei die Extrakte aus Aloe vera, Kamille, Algen, Rosmarin, Calendula, Ginseng, Gurke, Salbei, Brennnessel, Lindenblüten, Amika, und Hamamelis. Es können auch Mischungen aus zwei oder mehreren Pflanzenextrakten eingesetzt werden. Als Extraktionsmittel zur Herstellung der genannten Pflanzenextrakte können u.a. Wasser, Alkohole sowie deren Mischungen verwendet werden. Unter den Alkoholen sind dabei niedere Alkohole wie Ethanol und Isopropanol, aber auch mehnivertige Alkohole wie Ethylenglykol, Propylenglykol und Butylenglykol bevorzugt, und zwar sowohl als alleiniges Extraktionsmitttel als auch in Mischungen mit Wasser. Die Pflanzenextrakte können sowohl in reiner als auch in verdünnter Form eingesetzt werden.

Kosmetische Zubereitungen, die erfindungsgemäß, synergistische Mischungen von 1,2-Alkandiolen enthalten, können zumal wenn kristalline oder mikrokristalline Festkörper, beispielsweise anorganische Mikropigmente in die Zubereitungen eingearbeitet werden sollen, auch anionische, kationische, nichtionische und/oder amphotere Tenside enthalten. Tenside sind amphiphile Stoffe, die organische, unpolare Substanzen in Wasser lösen können. Bei den hydrophilen Anteilen eines Tensidmoleküls handelt es sich dabei meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch. Typisch für nicht-ionische Tenside sind Polyether-Ketten. Nichtionische Tenside bilden in wäßrigem Medium keine Ionen.

### A. Anionische Tenside

Vorteilhaft zu verwendende anionische Tenside sind Acylaminosäuren (und deren Salze), wie
- Acylglutamate, beispielsweise Natriumacylglutamat, Di-TEA-palmitoylaspartat und Natrium Capryl/ Capringlutamat,
- Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
- Sarcosinate, beispielsweise Myristoyl Sarcosin, TEA-Iauroytsarcosinat, Natriumlauroylsarcosinat und Natriumcocoyisarkosinat,
- Taurate, beispielsweise Natriumlauroyltaurat und Natriummethylcocoyltaurat,
- Acyllactylate, Lauroyllactylat, Caproyllactylat
- Alaninate
   Carbonsäuren und Derivate, wie
- beispielsweise Laurinsäure, Aluminiumstearat, Magnesiumalkanolat und Zinkundecylenat,
- Ester-Carbonsäuren, beispielsweise Calciumstearoyllactylat, Laureth-6 Citrat und Natrium PEG-4 Lauramidcarboxylat,
- Ether-Carbonsäuren, beispielsweise Natriumlaureth-13 Carboxylat und Natrium PEG-6 Cocamide Carboxylat,
   Phosphorsäureester und Salze, wie beispielsweise DEA-Oleth-10-Phosphat und Dilaureth-4 Phosphat, Sulfonsäuren und Salze, wie
- Acyl-isothionate, z.B. Natrium-/ Ammoniumcocoyl-isethionat,
- Alkylarylsulfonate,
- Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsutfat,
- Sulfosuccinate, beispielsweise Dioctyinatriumsulfosuccinat, Dinatriumlaureth-sulfosuccinat, Dinatriumlaurylsulfosuccinat und Dinatriumundecylenamido MEA-Sutfosuccinat
   sowie
   Schwefelsäureester, wie
- Alkylethersulfat, beispielsweise Natrium-, Ammonium-, Magnesium-, MIPA, TIPA-Laurethsulfat, Natriummyrethsulfat und Natrium C12-13 Parethsulfat,
- Alkylsulfate, beispielsweise Natrium-, Ammonium- und TEA- Laurylsulfat.

### B. Kationische Tenside

Vorteilhaft zu verwendende kationische Tenside sind
- Alkylamine,
- Alkylimidazole,
- Ethoxylierte Amine und
- Quatemäre Tenside.
   RNH₂CH₂CH₂COO⁻ (bei pH=7)
   RNHCH₂CH₂COO- B⁺ (bei pH=12) B⁺ = beliebiges Kation, z.B. Na⁺
- Esterquats

Quatemäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkylamidopropylhydroxysulfain. Die verwendeten kationischen Tenside können ferner bevorzugt gewählt werden aus der Gruppe der quaternären Ammoniumverbindungen, insbesondere Benryltrialkyl-ammoniumchloride oder -bromide, wie beispielsweise Benzyldimethylstearyl-ammoniumchlorid, ferner Alkyltrialkylammoniumsalze, beispielsweise Cetyltrimethylammoniumchlorid oder -bromid, Alkyldimethylhydroxy-ethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, lmidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- Acyl-/dialkylethylendiamin, beispielsweise Natriumacylamphoacetat, Dinatrium-acylamphodipropionat, Dinatriumalkylamphodiacetat, Natriumacylamphohydroxy-propytsulfonat, Dinatriumacylamphodiacetat und Natriumacylamphopropionat,
- N-Alkylaminosäuren, beispielsweise Aminopropylalkylglutamid, Alkylaminopro-pionsäure, Natriumalkylimidodipropionat und Lauroamphocarboxyglycinat.

### D. Nicht-ionische Tenside

Vorteilhaft zu verwendende nicht-ionische Tenside sind
- Alkohole,
- Alkanolamide, wie Cocamide MEA/ DEA/ MIPA,
- Aminoxide, wie Cocoamidopropylaminoxid,
- Ester, die durch Veresterung von Carbonsäuren mit Ethylenoxid, Glycerin, Sorbitan oder anderen Alkoholen entstehen,
- Ether, beispielsweise ethoxylierte/propoxylierte Alkohole, ethoxylierte/ propoxylierte Ester, ethoxylierte/ propoxylierte Glycerinester, ethoxylierte/ propoxy-lierte Cholesterine, ethoxylierte/ propoxylierte Triglyceridester, ethoxyliertes prop-oxyliertes Lanolin, ethoxylierte/ propoxylierte Polysiloxane, propoxylierte POE-Ether und Alkylpolyglycoside wie Laurylglucosid, Decylglycosid undCocoglyco-sid.
- Sucroseester, -Ether
- Polyglycerinester, Diglycerinester, Monoglycerinester
- Methylglucosester, Ester von Hydroxysäuren

Vorteilhaft ist ferner die Verwendung einer Kombination von anionischen und/ oder amphoteren Tensiden mit einem oder mehreren nicht-ionischen Tensiden. Die oberflächenaktive Substanz kann in einer Konzentration zwischen 1 und 98 Gew.% in den erfindungsgemäßen, synergistische Mischungen von 1,2-Alkandiolen enthaltenden Zubereitungen vorliegen, bezogen auf das Gesamtgewicht der Zubereitungen.

Kosmetische oder dermatologische Zubereitungen, die erfindungsgemäße synergistische Mischungen von 1,2-Alkandiolen enthalten, können auch als Emulsionen vorliegen.

Die Ölphase kann vorteilhaft gewählt werden aus folgender Substanzgruppe:
- Mineralöle, Mineralwachse
- fette Öle, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Vorteilhaft einsetzbar sind (a) Ester aus gesättigten und/oder ungesättigten verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, (b) Ester aus aromatischen Carbonsäuren und gesättigten und/oder ungesättigten, verzweigten und/oder unverzweigten Alkoholen einer Kettenlänge von 3 bis 30 C-Atomen. Bevorzugte Esteröle sind Isopropylmyristat, Isopropylpalmitat, Isopropylstearat, Isopropyloleat, n-Butylstearat, n-Hexyllaurat, n-Decyloleat, Isooctylstearat, Isononylstearat, Iso-nonylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr. Auch beliebige Abmischungen solcher Öl- und Wachskomponenten sind vorteilhaft einsetzbar. In manchen Fällen ist es auch vorteilhaft, Wachse, beispielsweise Cetylpalmitat, als alleinige Lipidkomponente der Ölphase einzusetzen, vorteilhaft wird die Ölphase gewählt aus der Gruppe , die besteht aus 2-Ethylhexylisostearat, Octyldodecanol, lsotridecylisononanoat, lsoeicosan, 2-Ethythexylcocoat, C₁₂₋₁₅-Alkylbenzoat, Capryl-Caprinsäuretriglycerid und Dicaprylylether. Besonders vorteilhaft sind Mischungen aus C₁₂₋₁₅₋Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und lsotridecylisononanoat. Auch die Kohlenwasserstoffe Paraffinöl, Squalan und Squalen lassen sich vorteilhaft verwenden. Vorteilhaft kann die Ölphase ferner einen Gehalt an cyclischen oder linearen Silikonölen aufweisen oder vollständig aus solchen Ölen bestehen, wobei es allerdings bevorzugt ist, außer dem Silikonöl oder den Silikonölen einen zusätzlichen Gehalt an anderen Ölphasenkomponenten zu verwenden. Cyclomethicon (z.B. Decamethylcyclopentasiloxan) kann vorteilhaft als Silikonöl eingesetzt werden. Aber auch andere Silikonöle sind vorteilhaft verwendbar, beispielsweise Undecamethylcyclotrisiloxan, Polydimethylsiloxan und Poly(methylphenylsiloxan). Besonders vorteilhaft sind ferner Mischungen aus Cyclomethicon und lsotridecylisononanoat sowie aus Cyclomethicon und 2-Ethythexylisostearat.

Die wäßrige Phase von als Emulsion vorliegende Zubereitungen, die erfindungsgemäß synergistische Mischungen von 1,2-Alkandiolen enthalten , kann umfassen: Alkohole, Diole oder Polyole niedriger C-Zahl, sowie deren Ether, vorzugsweise Ethanol, lsopropanol, Propylenglykol, Glycerin, Ethylenglykol, Ethylenglykol- monoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Als Emulsion vorliegende Zubereitungen, die erfindungsgemäße, synergistische Mischungen von 1,2-Alkandiolen enthalten, umfassen vorteilhaft einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-)ₙ-H,
- der veretherten Fettsäureethoxylate der allgemeinen Formel

   R-COO-(-CH₂CH₂-O-)ₙ-R',

   der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-COO-(-CH₂-CH₂O)ₙ-OOH,

   und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H
- der Polypropylenglycolether der allgemeinen Formel

   R-O-(-CH₂₋CH(CH₃)-O-)ₙ₋R'
- der propoxylierten Wollwachsalkohole,
- der veretherten Fettsäurepropoxylate R-COO-(-CH₂-CH(CH₃)-O)ₙ-R'
- der veresterten Fettsäurepropoxylate der allgemeinen Formel

   R-COO-(-CH₂CH(CH₃)-O-ₙC(O)-R'
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂CH(CH₃)-O-)ₙCH₂-COOH,
- der Alkylethersulfate bzw. die diesen Sulfaten zugrundeliegenden Säuren der allgemeinen Formel R-O-(-CH₂-CH(CH₃)-O-)ₙ-SO₃-H,
- der Fettalkoholethoxylatelpropoxylate der allgemeinen Formel R-O-Xₙ-Yₘ-H
- der Polypropylenglycolether der allgemeinen Formel R-O-Xₙ-Yₘ-R'
- der veretherten Fettsäutepropoxylate der allgemeinen Formel R-COO-Xₙ-Yₘ-R'
- der Fettsäureethoxylatelpropoxylate der allgemeinen Formel R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen lsoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearyfalkohote, Cetylalkohole, Cetylstearylalkohole (Cetearylalkohole) zu
wählen. Insbesondere bevorzugt sind:
Polyethylenglycol(13)stearylether (Steareth-13),
Polyethylenglycol(14)stearylether (Steareth-14),
Polyethylenglycol(15)stearylether (Steareth-15),
Polyethylenglycol (16)stearylether (Steareth-16),
Polyethylenglycol(17)stearylether (Steareth-17),
Polyethylenglycol-(18)stearylether (Steareth-18),
Polyethylenglycol(19)stearylether (Steareth-19),
Polyethylenglycol(20)stearylether (Steareth-20),
Polyethylenglycol(12)isostearylether (Isosteareth- 12),
Polyethylenglycol(13)isostearylether (Isosteareth-13),
Polyethylenglycol(14)isostearylether (Isosteareth-14),
Polyethylenglycol(15)isostearylether (Isosteareth-15),
Polyethylenglycol(16)isostearylether (Isosteareth-16),
Polyethylenglycol(17)isostearylether (Isosteareth-17),
Polyethylenglycol-(18)isostearylether (Isosteareth-18),
Polyethylenglycol(19)isostearylether (Isosteareth-19),
Polyethylenglycol(20)isostearylether (Isosteareth-20),
Potyethylenglycol(13cetylether (Ceteth-13),
Polyethylenglycol (14)cetylether (Ceteth-14),
Polyethylenglycol(15)cetylether (Ceteth-15),
Polyethylengtycol(16)cetylether (Ceteth-16),
Polyethylenglycol(17)cetylether (Ceteth-17),
Polyethylenglycol(18)cetyiether (Ceteth-18),
Polyethylenglycol(19)cetylether (Ceteth-19),
Polyethylenglycol(20)cetylether (Ceteth-20),
Polyethylenglycol(13)isocetylether (Isoceteth-13),
Polyethylenglycol(14)isocetylether (Isoceteth-14),
Polyethylenglycol(15)isocetylether (Isoceteth-15),
Polyethylenglycol(16)isocetylether (lsoceteth-16),
Polyethylenglycol(17)isocetylether (Isoceteth-17),
Polyethylenglycol(18)isocetylether (Isoceteth-18),
Polyethylenglycol(19)isocetylether(Isoceteth-19),
Polyethylenglycol(20)isocetylether (Isoceteth-20),
Polyethylenglycol(12)oleylether (Oleth-12),
Polyethylenglycol(13)oleylether (Oleth-13),
Polyethylenglycol(14)oleylether (Oleth-14),
Polyethylenglycol(15)oleylether (Oleth-1 5),
Polyethylenglycol(12)laurylether (Laureth-12),
Polyethylenglycol(12)isolaurylether (Isolaureth 12).
Polyethylenglycol(13)cetylstearylether (Ceteareth-13),
Polyethylenglycol(14)cetylstearylether (Ceteareth-14),
Polyethylenglycol(15) ketylstearylether (Ceteareth-15),
Polyethylenglycol (16) cetytstearylether (Ceteareth-16),
Polyethylenglycol (17) cetylstearylether (Ceteareth-17),
Polyethylenglycol(18) cetylstearylether (Ceteareth-18),
Polyethylenglycol-(19)cetylstearylether (Ceteareth-19),
Polyethylenglycol (20)cetylstearylether (Ceteareth-20),

Es ist ferner von Vorteil, die Fettsäureethoxylate aus folgender Gruppe zu wählen: Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethytengtycol(12)isostearat, Polyethylenglycol(13)isostearat,
Polyethylenglycol-(14)isostearat, Polyethylenglycol(15)isostearat,
Polyethylenglycot(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylengiycoi-(22)isostearat, Polyethylenglycoi(23)isostearat,
Polyethylenglyool(24)isostearat, Polyethylenglycol(25)isostearat,
Polyethylenglycot(12)oleat, Potyethylenglycol(13)oleat,
Polyethylenglycol(14)oleat, Polyethylenglycot(15)oleat,
Polyethylenglycol(16)oleat, Polyethylenglycol(17)oleat,
Polyethylenglycol(18)oleat, Polyethylenglycol(19)oleat,
Polyethylenglycol(20)oleat

Als ethoxylierte Alkylethercarbonsäure bzw. deren Salz kann vorteilhaft das Natriumlaureth-11-carboxylat verwendet werden. Als Alkylethersulfat kann Natrium Laureth 1-4 sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllautat, Polyethylenglycol(6)glycerylcaprat/caprinat, Polyethylenglycol(20)glyceryloleat, Polyethylenglycol(20)glycerylisostearat,Polyethylenglycol(18)glyceryloleat/cocoat zu wählen.

Es ist ebenfalls günstig, die Sorbitanester aus der Gruppe
Polyethylenglycol(20)sorbitanmonolaurat,
Polyethylenglycot(20)sorbitanmonostearat,
Polyethylenglycol(20)sorbitanmonoisostearat,
Polyethylenglycoi(20)sorbftanmonopaimftat,
Polyethylenglycoi(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 -18 C-Atomen.

Insbesondere vorteilhafte W/O-Emulgatoren sind Glycerylmonostearat, Glycerylmonoisostearat, Glycerylmonomyristat, Glycerylmonooleat, Diglyceryimonostearat, Diglycerylmonoisostearat, Propylenglycolmonostearat, Propylenglycalmonoisostearat, Propylenglycolmonocaprylat, Propylenglycolmonolaurat, Sorbitanmonoisostearat, Sorbitanmonolaurat, Sorbitanmonocaprytat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycoi(2)stearylether(Steareth-2), Glycerytmonolaurat, Glycerylmonocaprinat, Glycerylmonocaprylat.

Die Erfindung wird nachfolgend anhand von Beispielen unter Bezugnahme auf die beigefügte Figur näher erläutert:

### Beispiel 1: Prüfung der ausreichenden Konservierung durch synergistisch wirksame 1,2-Diol-Gemische

Eine Prüfung auf ausreichende Konservierung erfolgte gemäß Europäischem Arzneibuch.

Die Prüfung besteht somit aus der Kontamination der Zubereitung, wenn möglich in ihrem Endverhältnis, mit einem vorgeschriebenen lnokulum geeigneter Mikroorganismen, der Lagerung der beimpften Zubereitung bei einer bestimmten Temperatur, der Entnahme der Proben aus dem Behältnis in bestimmten Zeitabständen und der Bestimmung der Anzahl der Mikroorganismen in den so entnommenen Proben. Die konservierenden Eigenschaften sind ausreichend, wenn sich unter den Bedingungen der Prüfung eine eindeutige Verminderung oder gegebenenfalls keine Vermehrung der Keimzahl in den beimpften Zubereitungen nach den vorgeschriebenen Zeiten bei den vorgeschriebenen Temperaturen ergibt. Experimentelle Details der Versuchsdurchführung sind im Europäischen Arzneibuch (ISBN 3-7692-2768-9; Nachtrag 2001 zur 3. Ausgabe, Seite 421-422, Kapitel 5.1.3) beschrieben.

### Testkeime:

Folgende Mikroorganismen-Stämme wurden für die Tests auf ausreichende Konservierung verwendet:
A: *Escherichia coli* ATCC 8739
B: *Pseudomonas aeruginosa* ATCC 9027
C: *Staphylococcus aureus* ATCC 6538
D: *Candida albicans* ATCC 10231
E: *Aspergillus niger* ATCC 16404

Die anfängliche Keimzahl (KBE/g; "0"-Wert") lag bei den unterschiedlichen Testreihen im Bereich von 240 000 bis 300 000.

### Formulierung:

Für die Tests auf ausreichende Konservierung wurden (a) in Betracht gezogene, potentiell synergistisch wirksame Gemische, sowie (b) zu Vergleichszwecken die entsprechenden unvermischten 1,2-Diole in O/W-Emulsionen eingearbeitet.

Tabelle 2 zeigt beispielhaft die Rezeptur für ein erfindungsgemäßes Gemisch aus 1,2-Hexandiol (2%) und 1,2-Octandiol (1%). Die Variation der Gesamtkonzentration an 1,2-Diolen oder 1,2-Diolgemischen in den 0/W-Emulsionen wurde durch Erhöhung bzw. Erniedrigung des Wasseranteils ausgeglichen, so dass ansonsten identische Rezepturen in Summe immer 100 Gewichtsteile umfassten.

**Tabelle 2: Beispielformulierung für eine O/W-Emulsion enthaltend ein 1,2-Hexandiol (2%)/1,2-0ctandiol (1%)-Gemisch**

| **Rohstoff (z.T. Handelsname angegeben)** | **Hersteller** | **Gew.-%** |
|---|---|---|
| | | |
| Wasser, keimarm | | 76,1 |
| Citronens. 10% ig | | 0,4 |
| Dragophos S | DRAGOCO | 2 |
| PCL Liquid | DRAGOCO | 3 |
| Isodragol | DRAGOCO | 7 |
| Lanette 18 | COGNIS | 4,5 |
| Dracorin GMS | DRAGOCO | 2 |
| Dow Corning 200 fluid | Dow Coming | 2 |
| 1,2-Hexandiol | | 2 |
| 1,2-Octandiol | | 1 |
| | | |
| Summe | | 100 |

### Ergebnis:

Die Ergebnisse der Konservierungsmittel-Belastungstests zu den untersuchten 1,2-Diolen und 1,2-Diol-Gemischen sind in Tabelle 3 dargestellt. Überraschenderweise zeigte sich, dass 1,2-Diolgemische bestehend aus definierten Mengenverhältnissen an 1,2-Pentandiol, 1,2-Hexandiol, 1,2-Octandiol und/oder 1,2-Decandiol eine weitaus höhere Wirksamkeit aufweisen als die in gleich hoher Konzentration dosierten Einzelsubstanzen. Dies zeigt sich vor allem in den nach 28 Tagen verbleibenden Rest-Keimzahlen. Besonders wirksam erwiesen sich hierbei binäre und ternäre Gemische von 1,2-Hexandiol mit 1,2-Pentandiol und/oder 1,2-Octandiol. Die Konzentration koloniebildender Einheiten (KBE) konnte bei allen 5 Testkeimen (*Escherichia coli, Pseudomonas aeruginosa, Staphylococcus aureus, Candida albicans und Aspergillus niger*) im gewählten Testzeitraum auf den bevorzugt anzustrebenden 0-Wert reduziert werden.

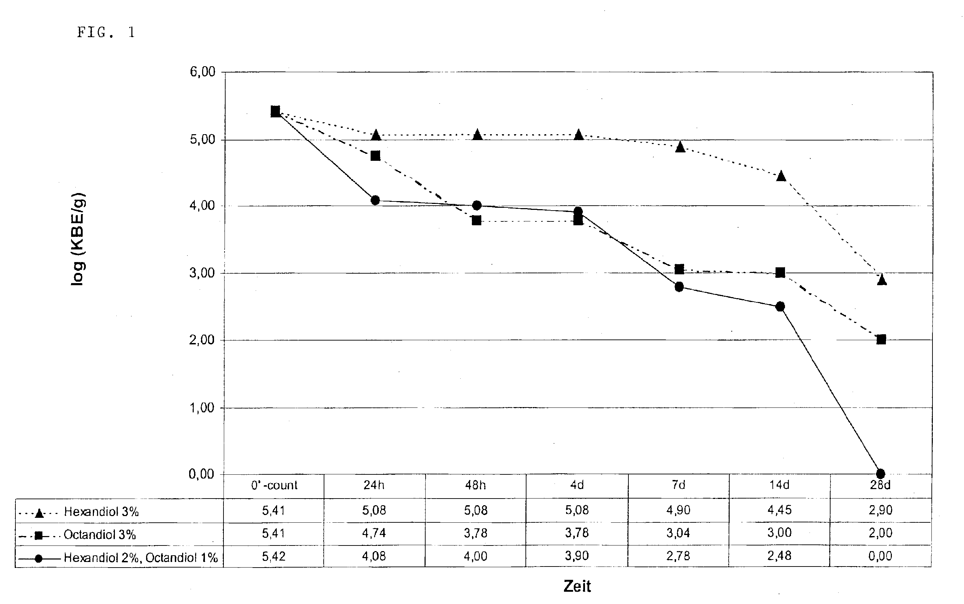

Wie aus Tabelle 3 sowie Fig. 1 (Test auf ausreichende Konservierung für *Aspergillus niger* über einen Zeitraum von 28 Tagen; logarithmische Darstellung der Keimzahlreduktion für 1,2-Hexandiol (3% in O/W-Emulsion), für 1,2-Octandiol (3%) sowie für ein 1,2-Hexandiol/1,2-Octandiol-Gemisch (Mengenverhältniss 2:1; Dosierung ebenfalls 3%) ersichtlich ist, konnte bei *Aspergillus niger,* einem hinsichtlich der Konservierung industrieller Produkte besonders problematischen Keim, die Keimzahl durch Verwendung der erfindungsgemäßen Mischungen innerhalb von 28 Tagen auf 0 reduziert werden. Die zu Vergleichszwecken ebenfalls in einer Dosierung von 3% getesteten Einzelsubstanzen (1,2-Pentandiol, 1,2-Hexandiol, 1,2-Octandiol und 1,2-Decandiol; Tabelle 3) ermöglichten bei *Aspergillus niger* hingegen keine Reduktion der Anzahl koloniebildender Einheiten (KBE) bis auf den gewünschten 0-Wert. Die Testreihen (Fig. 1 und Tab. 3) zeigen somit beispielhaft, dass 1,2-Diolgemische bestehend aus mindestens zwei unterschiedlichen Mitgliedem der Gruppe, die aus 1,2-Pentandiol, 1,2-Hexandiol, 1,2-Octandiol und 1,2-Decandiol besteht, eine synergistisch verstärkte Wirksamkeit besitzen.

Die synergistische Wirksamkeitsverstärkung der erfindungsgemäßen Diol-Gemische lässt sich basierend auf den vorliegenden Daten auch anhand der Gleichung von Kull (F.C. Kull et al.; Applied Miaobiology Vol. 9, p. 538-541 (1961); David C. Steinberg; Cosmetics & Toiletries Vol. 115 (No. 11), p. 59-62; November 2000; zur Berechnungsmethode siehe auch Tabelle 4) beweisen. Die Kull'sche Gleichung gestattet es, die Reinsubstanzen und die daraus hergestellten Wirkstoffmischungen hinsichtlich Ihrer antimikrobiellen Wirksamkeit zu vergleichen. Bestimmt wird hierbei der sogenannte Synergie-Index (SI), der ein Maß für eine synergistische, aber auch für eine eventuell antagonistische Wirksamkeit eines antimikrobiell wirksamen Gemisches darstellt. Ein synergistischer Effekt ist evident, wenn der ermittelte SI-Wert kleiner 1 ist. Errechnet sich ein SI von exakt 1 liegt hingegen ein rein additiver Effekt von zwei antimikrobiell wirksamen Substanzen vor. Bei einem SI-Wert größer als 1 liegt hingegen ein (häufig unerwünschter) antagonistischer Effekt vor.

Nachfolgend ist beispielhaft die Berechnung des SI-Wertes für die Behandlung von *Aspergillus niger* mit einer Mischung aus 1,2-Hexandiol und 1,2-Octandiol (Verhältnis 2:1) nach einer Inkubationsphase von 14 Tagen dargestellt (Tabelle 4) . Der berechnete SI von 0,106 zeigt deutlich, dass ein 2:1-Gemisch von 1,2-Hexandiol und 1,2-Octandiol eine stark synergistische Wirkstoffkombination darstellt. Der 28-Tage SI-Wert konnte nicht ermittelt werden, da nach dieser Inkubationsphase die Keimzahlen bei Verwendung der Diol-Mischung bei 0 lagen (vergleiche Tabelle 3). In diesem Sonderfall lässt sich die Kull'sche Gleichung nicht anwenden; der Synergismus ist aber natürlich aufgrund der Keimzahl 0 ohnehin evident.

**Tabelle 4: Berechnung des Synergie-Index (SI) für 1,2-Hexandiol/1,2-Octandiol (Massenverhältnis: 2:1; Dosierung in O/W-Emulison: 3%; Testkeim: Aspergillus niger)**

| | A | B | C |
|---|---|---|---|
| | 1,2-Hexandiol | 1,2-Octandiol | 1,2-Hexandiol + 1,2-Octandiol; Massenverhältnis: 2:1 |
| Aspergillus niger: 14 Tage [KBE/ml) | 28000 | 1000 | 300 |
| | | | |
| Kull's Gleichung: SI = CxD/A + CxE/B | | | |
| | | | |
| A: Keimzahl für Substanz A | 28000 | | |
| B: Keimzahl für Substanz B | 1000 | | |
| C: Keimzahl für A + B Gemisch | 300 | | |
| D: Menge von A in C | 0,66 | | |
| E: Menge von B in C | 0,33 | | |
| **Sl: Synergie Index** | **0,106** | | |
| | | | |
| Literatur: Synergie Index: | | | |
| D.C.Steinberg; Cosmetics & Toiletries 115 (11); p. 59-62 (2000) | | | |
| F.C.Kull et al.; Applied Microbiology 9; p. 538-541 (1961) | | | |

### Beispiel 2: Ermittlung der Minimalen Hemmkonzentrationen gegenüber diversen Keimen und Berechnung von Synergie-Indices auf Basis von MHK-Werten

### Vorbemerkung:

Die Erkenntnis, dass die erfindungsgemäßen 1,2-Diolgemische über Ihre Verwendung als Konservierungsmittel hinaus auch zur Bekämpfung von Keimen geeignet sind, die z.B. für Körpergeruch verantwortlich sind, geht auf Untersuchungsreihen zurück, in denen die besonders relevanten Keime *Staphylococcus epidermidis, Corynebacterium xerosis* und *Brevibacterium epidermidis* untersucht wurden. Hierbei wurden neben den MHK-Werten für *Staphylococcus epidermidis, Corynebacterium xerosis* und *Brevibacterium epidermidis* ebenfalls die entsprechenden Synergie-Indices der erfindungsgemäßen, synergistisch wirksamen Gemische bestimmt (vergleiche Tabelle 7).

Darüber hinaus zeigten die MHK-Bestimmungen, dass die beanspruchten 1,2-Diolgemische auch gegenüber weiteren Testkeimen wie *Trichophyton mentagrophytes, Epidermophyton floccosum, Propionibacterium acnes* überraschend gut wirksam sind, wodurch die beanspruchten Diolgemische auch als Mittel gegen Mykosen oder Akne Einsatz finden können.

Lediglich gegenüber *Malassezia furfur* ergab sich ein antagonistischer Effekt.

### Allgemeine Testbedingungen:

Der Nachweis der antimikrobiellen Wirkung der reinen 1,2-Diole sowie der erfindungsgemäßen Diol-Gemische erfolgte mit Hilfe des Agardilutionsverfahrens in Anlehnung an DIN 58 940/ICS und DIN 58 944/ICS. Es wurden Petrischalen von 9,0 cm Durchmesser mit 13,5 ml frisch hergestelltem und bei 50 °C flüssig gehaltenem Mueller-Hinton-Agar (Merck, Art. 1.05437 bzw. Wilkins-Chalgren-Agar-Boillon, Oxoid, Art. CM 643, supplementiert mit 10 g Agar-Agar/Liter) beschickt, denen die verschiedenen Konzentrationen der verdünnten Proben in 10 Vol% = 1,5 ml zugesetzt wurden. Für den Testkeim *Malassezia furfur* wurde Mueller-Hinton-Agar verwendet, der 3% Tween80 (Merck, Art. 8.22 187) enthielt.

Jeweils 5 ml der Proben wurden in Aqua dest. verdünnt und auf 10 ml aufgefüllt. Durch fortlaufende 1:2-Verdünnung dieses Ansatzes mit Aqua dest., wurden die weiteren Testkonzentrationen der jeweiligen Verdünnungsreihen, die in Form geometrischer Reihen angelegt wurden, hergestellt.

Durch eine weitere Verdünnung mit dem Testagar (1,5 ml Probe bzw. entsprechender Verdünnungen + 13,5 ml Agar) wurden jeweils 10-fach niedrigere Endkonzentrationen erreicht (entspricht einer Anfangskonzentration von jeweils 50000 ppm). Pro Testkonzentration und Nährmedium wurden zwei Agarplatten gegossen.

Es wurden folgende Kontrollen mit jeweils zwei Agarplatten durchgeführt:

| | | |
|---|---|---|
| K1: | 15,0 ml Mueller-Hinton-Agar | (unbeimpft) |
| K2: | 13,5 ml Mueller-Hinton-Agar + 1,5 ml Aqua dest | (unbeimpft) |
| K3: | 13,5 ml Mueller-Hinton-Agar + 1,5 ml Aqua dest | (beimpft) |
| K4: | 15,0 ml Mueller-Hinton-Agar | (beimpft) |
| K5: | 15,0 ml Muelter-Hinton-Agar + 3% Tween 80 | (unbeimpft) |
| K6: | 13,5 ml Mueller-Hinton-Agar + 3% Tween 80 + 1,5 ml Aqua dest. | (unbeimpft) |
| K7: | 13,5 ml Mueller-Hinton-Agar + 3% Tween 80 + 1,5 ml Aqua dest. | (beimpft) |
| K8: | 15,0 ml Mueller-Hinton-Agar + 3% Tween 80 | (beimpft) |
| K9: | 15,0 ml Wilkins-Chalgren-Agar | (unbeimpft) |
| K10: | 13,5 ml Wilkins-Chalgren Agar + 1,5 ml Aqua dest. | (unbeimpft) |
| K11: | 13,5 ml Wilkins-Chalgren Agar + 1,5 ml Aqua dest. | (beimpft) |
| K12: | 15,0 ml Wilkins-Chalgren Agar + 1,5 ml Aqua dest. | (beimpft) |

Nach Verfestigung und Trocknung (ca. 1 h bei 37 °C) wurden die Testplatten punktförmig mit jeweils 1 µl der in den nachfolgend aufgeführten Testkeimsuspensionen beimpft. Zur Überprüfung von Reinheit und Identität wurden die aerob wachsenden Bakterien (*Brevibacterium epidermidis, Corynebacterium xerosis, Staphylococcus epidermidis*) auf Columbia Blut-Agar (BioMerieux, Art. 43049). Der Schimmelpiz *Aspergillus niger,* die Hefe *Candida albicans* und die beiden Hautpilze *Trichophyton mentagrophytes und Epidermophyton floccosum* wurden auf Sabouraud-Agar (BioMörieux, Art. 43555) kultiviert. *Malassezia furfur* wurde auf Sabourad-HLT-Agar mit Enthemmern (Zusatz von 3% Tween80: 1%; Lecithin: 0,3%; Histidin: 0,1%; Merck, Art. 1.18368) angezüchtet. *Propionibacterium acnes* wurde auf Schaedler-Agar (BioMerieux, Art. 43273) kultiviert. Weitere Angaben zu den Testkeimen sind Tabelle 5 zu entnehmen.

**Tabelle 5: Testkeime (Stammbezeichnungen) und Keimzahlen**

| Testkeim | Stammbezeichnung | KBE*/ml |
|---|---|---|
| | | |
| *Brevibacterium epidermidis* | ATCC 35514 | 2,8 x 10⁷ |
| *Corynebacterium xerosis* | ATCC 7711 | 2,1 x 10⁷ |
| *Propionibacterium acnes* | ATCC 11829 | 2,0 x 108 |
| *Staphylococcus epidermidis* | ATCC 12228 | 2,2 x 107 |
| *Malassezia furfur* | DSM 6171 | 3,0 x 10⁷ |
| *Epidermophyton floccosum* | CBS 55384 | 2,1 x 10⁷ |
| *Trichophyton mentagrophytes* | CBS 26379 | 3,3 x 10⁷ |
| | | |
| KBE*=koloniebildende Einheiten | | |
| | | |

Die Herstellung der Testkeimsuspensionen der aerob wachsenden bakteriellen Keime erfolgte durch Bebrütung von Mueller-Hinton-Bouillon (Merck, Art. 1.10293) bei 36 °C, die mit wenigen Einzelkolonien der jeweiligen Testkeime beimpft worden war. Nach dem Erreichen einer deutlichen Trübung wurde den Suspensionen so viel sterile Nährbouillon zugegeben, dass deren Trübung dem McFarland Standard 0,5 entsprach (ca. 1,5 x 10⁸ KBE/ml).

Zur Herstellung der übrigen Testkeimsuspensionen wurden die Teststämme auf den oben genannten, festen Nährmedien kultiviert, mittels sterilem Tupfer abgeerntet und in so viel Mueller-Hinton-Bouillon aufgenommen bzw. verdünnt, dass die Trübung der Suspensionen dem McFarland Standard 0,5 entsprach.

Alle Testkeimsuspensionen, mit Ausnahme von *Propionibacterium acnes,* wurden nochmals mit steriler Bouillon 1:10 verdünnt und deren Keimzahl im Oberflächenverfahren per Spiralometer ermittelt (Ergebnisse: siehe Tabelle 5).

Die inokulierten Platten wurden unter den in Tabelle 6 angegebenen Bedingungen bebrütet und anschließend ausgewertet. Als MHK (Minimale Hemmkonzentration) wurde die niedrigste Wirkstoffkonzentration angesehen, bei der makroskopisch kein Wachstum vorhanden ist. Minimales, kaum sichtbares Wachstum oder wenige kleine Einzelkolonien wurden als Hemmung bewertet.

**Tabelle 6: Inokulation und Bebrütung**

| Testkeim | Stamm-Bez | Wachstumsbedingungen | Nährmedium | Bebrütung |
|---|---|---|---|---|
| | | | | |
| *Brevibacterium epidermidis* | ATCC 35514 | Aerob | Mueller-Hinton-Agar | 18h bei 36 °C |
| *Corynebacterium xerosis* | ATCC 7711 | Aerob | Mueller-Hinton-Agar | 18h bei 36 °C |
| *Propionibacterium acnes* | ATCC 11829 | Anaerob | Wilkins-Chalgren-Agar | 72h bei 30 °C |
| *Staphylococus epidermidis* | ATCC 12228 | Aerob | Mueller-Hinton-Agar | 18h bei 36 °C |
| *Malassezia furfur* | DSM 6171 | Aerob | Mueller-Hinton-Agar + 3% Tween 80 | 72h bei 30 °C |
| *Trichophyton mentagrophytes* | CBS 26379 | Aerob | Mueller-Hinton-Agar | 72h bei 30 °C |
| *Epidermophyton Floccosum* | CBS 55384 | Aerob | Mueller-Hinton-Agar | 18h bei 36 °C |

### MHK-Werte für 1,2-Hexandiol, 1,2-Octandiol und eine Mischung der beiden Diole im Verhältnis C6/C8 = 2:1

Die MHK-Werte für 1,2-Hexandiol, 1,2-Octandiol sowie für eine Mischung der beiden Diole im Mengenverhältnis 2 Teile Hexandiol zu 1 Teil Octandiol wurden gemäß den beschriebenen allgemeinen Testbedingungen bestimmt (vergleiche Tabelle 7)

**Tabelle 7:**

| MHK-Werte [ppm] für 1,2-Hexandiol, 1,2-Octandiol und für ein C6/C8-Diolgemisch (2:1) | | | | | |
|---|---|---|---|---|---|
| Bestimmung der **Synergie-Indices (Sl)** gemäß Gleichung von Kull et al. ^{1,2} | | | | | |
| | | | | | |

| Microorganismus | Strain-No. | MHK C6 | MHK C8 | MHK C6/C8 2:1 | **Sl: C6/C8; 2:1** |
|---|---|---|---|---|---|
| | | | | | |
| *Staphylococcus epidermidis* | ATCC 12228 | 25000 | 12500 | 6250 | **0,55** |
| *Corynebacterium xerosis* | ATCC 7711 | 12500 | 6250 | 6250 | **0,66** |
| *Brevibacterium epidermidis* | ATCC 35514 | 25000 | 3125 | 6250 | **0,83** |
| *Propionibacterium acnes* | ATCC 11829 | 25000 | 6250 | 3125 | **0,25** |
| *Malassezia furfur* | DSM 6171 | 12500 | 50000 | 50000 | **2,97** |
| *Trichophyton mentagrophytes* | CBS 26379 | 6300 | 1562 | 1562 | **0,49** |
| *Epidermophyton floccosum* | CBS 55384 | 6250 | 3125 | 1562 | **0,32** |
| | | | | | |
| ¹ F.C.Kull et al.; Applied Microbiology 9; p. 538-541 (1961) | | | | | |
| ² D.C.Steinberg; Cosmetics & Toiletries 115 (11); p. 59-62 (2000) | | | | | |

Die In Tabelle 7 dargestellten Ergebnisse zeigen beispielhaft für 1,2-Hexandiol und 1,2-Octandiol die synergistische Wirksamkeitsverstärkung des 2:1-Gemisches der beiden Diole. Auch Mikroorganismen wie *Staphylococcus epidermidis, Brevibacterium epidermidis, Corynebacterium xerosis, Propionibacterium acnes, Trichophyton mentagrophytes* und *Epidermophyton floccosum* werden demnach im direkten Vergleich zu den entsprechenden Einzelsubstanzen durch das 1,2-Diol-Gemisch deutlich stärker gehemmt. Die basierend auf den MHK-Werten mit Hilfe der Kull'schen Gleichung ermittelten Synergie Indices sind ebenfalls in Tabelle 7 dargestellt. Die SI-Werte zeigen deutlich, dass das 1,2-Hexandiol/1,2-Octandiol-Diolgemisch eine synergistisch verstärkte Wirksamkeit besitzt und neben seiner hervorragenden Wirksamkeit als Konservierungsmittel (vergleiche Beispiel 1) auch bevorzugt zur Bekämpfung von Körpergeruch (Sl *Staphylococcus epidermidis:* 0,55; Sl *Corynebacterium xerosis:* 0,66; Sl *Brevibacterium epidermidis:* 0,83), zur Bekämpfung von Akne (SI *Propionibacterium acnes: 0,25)* und zur Bekämpfung der durch *Trichophyton*- und *Epidermophyton*-Arten verursachten Haut- und Nagelkmykosen (SI *Trichophyton mentagrophytes:* 0,49; Sl *Epidermophyton floccosum:* 0,32) eingesetzt werden kann. Bei *Malassezia furfur* konnte hingegen für das 1,2-Hexandiol/1,2-Octandiol-Gemisch kein synergistischer Effekt aufgezeigt werden (Sl Malassezia furfur: 2,97, d.h. antagonistischer Effekt).

### Beispiel 3: Prüfung der ausreichenden Konservierung durch Gemische von (a) synergistischen Mischungen von 1,2-Alkandiolen mit (b) weiteren Konservierunctsmitteln

Die Prüfung auf ausreichende Konservierung durch Gemische von (a) synergistischen Mischungen von 1,2-Alkandiolen mit (b) weiteren Konservierungsmitteln erfolgte ebenfalls gemäß Europäischem Arzneibuch. Die Versuchsdurchführung ist in Beispiel 1 im Detail beschrieben.

### Testkeime:

Folgende Mikroorganismen-Stämme wurden für die Tests auf ausreichende Konservierung verwendet:
A: *Escherichia coli* ATCC 8739
B: *Pseudomonas aeruginosa* ATCC 9027
C: *Staphylococcus aureus* ATCC 6538
D: *Candida albicans* ATCC 10231
E: *Aspergillus niger* ATCC 16404

Die anfängliche Keimzahl (KBE/g; "0"-Wert") lag bei den unterschiedlichen Testreihen im Bereich von 280 000 bis 320 000.

### Formulierung:

Für die Tests auf ausreichende Konservierung wurden die in Betracht gezogenen, potentiell synergistischen Wirkstoffkombinationen bestehend aus (a) den erfindungsgemäßen 1,2-Diolgemischen und (b) weiteren Konservierungsmitteln, in definierter Menge in Emulsionen eingearbeitet. Zu Vergleichszwecken wurden die entsprechenden erfindungsgemäßen 1,2-Diolgemische bzw. die des weiteren geprüften Konservierungsmittel separat in die gleichen O/W-Emulsionen eingearbeitet.

Tabelle 8 zeigt beispielhaft die Rezeptur für ein erfindungsgemäßes Gemisch bestehend aus 0,05% Euxyl K400 (Mischung aus 1,2-Dibrom-2,4-dicyanbutan (20 %) und 2-Phenoxyethanol (80%)), 0,25% 1,2-Hexandiol und 0,25% 1,2-Octandiol. Die Variation der Gesamtkonzentration an 1,2-Diolgemisch bzw. zusätzlichem Konservierungsmittel Euxyl K400 in den ONV-Emulsionen wurde durch Erhöhung bzw. Erniedrigung des Wasseranteils ausgeglichen, so dass ansonsten identische Rezepturen in Summe immer 100 Gewichtsteile umfassten.

**Tabelle 8: Beispielformulierungen für O/W-Emulsion enthaltend A) 0,1% Euxyl K 400, B) 1% eines 1,2-Diolgemisches zusammengesetzt aus 0,5% 1,2-Hexandiol und 0,5% 1,2-Octandiol sowie C) eine Wirkstoffkombination bestehend aus 0,05% Euxyl K 400, 0,25% 1,2-Hexandiol und 0,25% 1,2-Octandiol.**

| **Rohstoff** | **Hersteller** | | **Gew.-%** | |
|---|---|---|---|---|
| **(z.T. Handelsname** | | **A** | **B** | **C** |
| **Angegeben)** | | **0,1% Euxyl K400** | **0,5% Hexandiol** | **0,05% Euxyl K400** |
| | | | **0,5% Octandiol** | **0,25% Hexandiol** |
| | | | | **0,25% Octandiol** |
| Octandiol | DRAGOCO | 0 | **0,5** | **0,25** |
| Hexandiol | DRAGOCO | 0 | **0,5** | **0,25** |
| Euxyl K 400 | Schülke/Mayr | **0,1** | 0 | **0,05** |
| K-Sorbat | Ringe+Kuhlma nn | 0 | 0 | 0 |
| Phenoxyethanol | | 0 | 0 | 0 |
| Parabenmischung | | 0 | 0 | 0 |
| Wasser, keimarm | | 79,25 | 78,35 | 78,75 |
| Citronens. 10% ig | | 0,15 | 0,15 | 0,2 |
| Dragophos S | DRAGOCO | 2 | 2 | 2 |
| | | | | |
| PCL Liquid | DRAGOCO | 3 | 3 | 3 |
| lsodragoi | DRAGOCO | 7 | 7 | 7 |
| Lanette 18 | COGNIS | 4,5 | 4,5 | 4,5 |
| Dracorin GMS | 2/008474 | 2 | 2 | 2 |
| Dow Coming 200 fl. | Dow Coming | 2 | 2 | 2 |
| | | | | |
| Summe | | 100 | 100 | 100 |

### Ergebnis:

Die Ergebnisse der Konservierungsmittel-Belastungstests zu den untersuchten Wirkstoffkombinationen bestehend aus einem erfindungsgemäßen synergistischen Gemisch von 1,2-Alkandiolen und einem weiteren Konservierungsmittel sind in den Tabellen 9 und 10 beispielhaft am System 1,2-Hexandiol/1,2-Octandiol/Euxyl K400 dargestellt. Überraschenderweise zeigte sich, dass nicht nur 1,2-Diolgemische selbst sondern auch Kombinationen von 1,2-Diolgemischen mit weiteren Konservierungsmitteln gegenüber den in gleich hoher Konzentration dosierten Einzelsubstanzen eine signifikante synergistische Wirksamkeitsverstärkung erzielen können. Im genannten Beispiel zeigt sich dies vor allem in den nach 28 Tagen verbleibenden Rest-Keimzahlen für Aspergillus niger. Wie aus Tabelle 9 ersichtlich ist, konnte bei *Aspergillus niger,* einem hinsichtlich der Konservierung industrieller Produkte besonders problematischen Keim, die Keimzahl durch Verwendung der Formulierung C innerhalb von 28 Tagen auf 2800 reduziert werden. Das zu Vergleichszwecken in einer Dosierung von 1% getestete Diolgemisch gemäß Formulierung B (1,2-Hexandiol + 1,2-Octandiol; Mengenverhältnis 1:1) und die ebenfalls zu Vergleichszwecken in einer Konzentration von 0,1% getestete Formulierung A (mit Euxyl K400) ermöglichten bei *Aspergillus niger* hingegen keine derart signifikante Reduktion der Anzahl koloniebildender Einheiten (KBE). Die Testreihe (Tab. 9) zeigt somit beispielhaft, dass Wirkstoffgemische bestehend aus (a) mindestens zwei unterschiedlichen Mitgliedem der Gruppe, die aus 1,2-Pentandiol, 1,2-Hexandiol, 1,2-Octandiol und 1,2-Decandiol besteht, und (b) einem weiteren Konservierungsmittel eine synergistische nochmals verbesserte Wirkung besitzen können.

**Tabelle 9: Prüfung auf ausreichende Konservierung für eine Wirkstoffkombination bestehend aus einem erfindungsgemäßen, synergistisch wirksamen Diolgemisch (1,2-Hexandiol und 1,2-Octandiol) sowie einem weiteren Konservierungsmittel Euxyl K400; [KBE/ml]**

| | | | | | |
|---|---|---|---|---|---|
| | | | | | |

| **1. O/W Emulsion mit Formulierung A (0,1% Euxyl K400)** | | | | | |
|---|---|---|---|---|---|
| | | | | | |

| **Tage** | **Escherichia** | **Pseudomonas** | **Staphylococcus** | **Candida** | **Aspergillus** |
|---|---|---|---|---|---|
| | **coli** | **aeruginosa** | **Aureus** | **albicans** | **niger** |
| 0 | 300.000 | 320.000 | 310.000 | 310.000 | 280.000 |
| 1 | 200 | 0 | 0 | <100 | 220.000 |
| 2 | <100 | 0 | 0 | <100 | 140.000 |
| 4 | 0 | 0 | 0 | 0 | 120.000 |
| 7 | 0 | 0 | 0 | 0 | 200.000 |
| 14 | 0 | 0 | 0 | 0 | 160.000 |
| 28 | 0 | 0 | 0 | 0 | 32.000 |
| | | | | | |

| **2. O/W Emulsion mti Formulierung B (0,5% Hexandiol + 0,5% Octandiol)** | | | | | |
|---|---|---|---|---|---|
| | | | | | |

| **Days** | **Escherichia** | **Pseudomonas** | **Staphylococcus** | **Candida** | **Aspergillus** |
|---|---|---|---|---|---|
| | **coli** | **aeruginosa** | **Aureus** | **albicans** | **niger** |
| 0 | 300.000 | 320.000 | 310.000 | 310.000 | 280.000 |
| 1 | 0 | 0 | 0 | 3.200 | 180.000 |
| 2 | 0 | 0 | 0 | 200 | 160.000 |
| 4 | 0 | 0 | 0 | 100 | 140.000 |
| 7 | 0 | 0 | 0 | 0 | 180.000 |
| 14 | 0 | 0 | 0 | 0 | 120.000 |
| 28 | 0 | 0 | 0 | 0 | 60.000 |
| | | | | | |

| **3. O/W Emulsion mit Formulierung C (0,25% Hexandiol + 0,25% Octandiol + 0,05% Euxyl K 400)** | | | | | |
|---|---|---|---|---|---|
| | | | | | |

| **Days** | **Escherichia** | **Pseudomonas** | **Staphylococcus** | **Candida** | **Aspergillus** |
|---|---|---|---|---|---|
| | **coli** | **aeruginosa** | **Aureus** | **albicans** | **niger** |
| 0 | 300.000 | 320.000 | 310.000 | 310.000 | 280.000 |
| 1 | <100 | <100 | <100 | <100 | 20.000 |
| 2 | 0 | 0 | 0 | 0 | 12.000 |
| 4 | 0 | 0 | 0 | 0 | 10.000 |
| 7 | 0 | 0 | 0 | 0 | 8.000 |
| 14 | 0 | 0 | 0 | 0 | 8.000 |
| 28 | 0 | 0 | 0 | 0 | 2.800 |

Die synergistische Wirksamkeitsverstärkung der Wirkstoffkombination bestehend aus (a) einer erfindungsgemäßen, synergistisch wirksamen Mischung von 1,2-Alkandiolen (z. B. 1,2-Hexandiol und 1,2-Octandiol) und (b) einem weiteren Konservierungsmittel (z. B. Euxyl K400) lässt sich ebenfalls basierend auf den vorliegenden Daten anhand der Gleichung von Kull beweisen (F.C. Kull et al.; Applied Microbiology Vol. 9, p. 538-541 (1961); David C. Steinberg; Cosmetics & Toiletries Vol. 115 (No. 11), p. 59-62; November 2000; zur Berechnungsmethode siehe auch Tabelle 10). Die Kull'sche Gleichung gestattet es, die Reinsubstanzen und die daraus hergestellten Wirkstoffmischungen hinsichtlich Ihrer antimikrobiellen Wirksamkeit zu vergleichen. Bestimmt wird hierbei der sogenannte Synergie-Index (SI), der ein Maß für eine synergistische, aber auch für eine eventuell antagonistische Wirksamkeit eines antimikrobiell wirksamen Gemisches darstellt. Ein synergistischer Effekt ist evident, wenn der ermittelte SI-Wert kleiner 1 ist. Errechnet sich ein Sl von exakt 1, liegt hingegen ein rein additiver Effekt von zwei antimikrobiell wirksamen Produkten vor. Bei einem Sl-Wert größer als 1 liegt hingegen ein (häufg unerwünschter) antagonistischer Effekt vor.

Nachfolgend ist beispielhaft die Berechnung des Sl-Wertes für die Behandlung von *Aspergillus niger* mit einer Mischung bestehend aus 1,2-Hexandiol, 1,2-Octandiol und Euxyl K400 (Formulierung C) nach einer Inkubationsphase von 28 Tagen dargestellt. Der berechnete Sl von 0,066 zeigt deutlich, dass die Mischung bestehend aus 1,2-Hexandiol, 1,2-Octandiol und Euxy K400 eine stark synergistische Wirkstoffkombination darstellt.

**Tabelle 10: Berechnung des Synergie-Index (SI) eines 1,2-Hexandiol/1,2-Octandiol/Euxyl K400-Gemisches (Formulierung C) bestehend zu gleichen Teilen aus dem Vergleichs-1,2-Alkandiolgemisch (Formulierung A) und der Vergleichslösung Euxyl K400 (Formulierung B) (Massenverhältnis 1:1.; Dosierung in O/W-Emulison: Testkeim: Aspergillus niger)**

| | A | B | C |
|---|---|---|---|
| | 1,2-Hexandiol 0,5% + 1,2-Octandiol 0,5% | Euxyl K400 0,1% | 1,2-Hexandiol (0,25%) + 1,2-Octandiol (0,25) + EuxylK400 (0,05%); |
| Aspergillus niger: 28 Tage [KBE/ml] | 60000 | 32000 | 2800 |
| | | | |
| Kull's Gleichung: Sl = CxD/A + CxE/B | | | |
| | | | |
| A: Keimzahl für Substanz A | 60000 | | |
| B: Keimzahl für Substanz B | 32000 | | |
| C: Keimzahl für A + B Gemisch | 2800 | | |
| D: Menge von A in C | 0,5 | | |
| E: Menge von B in C | 0,5 | | |
| **Sl: Synergie Index** | **0,066** | | |
| | | | |
| Literatur: Synergie Index: | | | |
| D.C.Steinberg; Cosmetics & Toiletries 115 (11); p. 59-62 (2000) | | | |
| F.C.Kull et al.; Applied Microbiology 9; p. 538-541 (1961) | | | |

**Tabelle3: Prüfung auf ausreichende Konservierung für 1,2- Diole und 1,2 Diolgemische [KBE/ml]**

| **SL** | **231001 PE3** | **231001 HE3** | **231001 OC3** | **010801 DE3** | **050601 DEH** | **231001 HE/OC** | **231001 HE/OC/PE** | **231001 HE/OC/DE** |
|---|---|---|---|---|---|---|---|---|
| **Code** | **PE3 Pentandiol 3%** | **HE3 Hexandlol 3%** | **OC3 Octandiol 3%** | **DE 3 Decandiol 3%** | **PE2DE1 Pentandiol 2% Decandiol 1%** | **HE2/OC1 Hexandiol 2% Octandiol 1%** | **PE1HE1O C1 Pentandiol 1% Hexandiol 1% Octandiol 1%** | **HE1OC1D E1 Hexandto) 1% Octandiol 1% Decandiol 1%** |
| **E.coli** | | | | | | | | |
| **0'-count** | 300.000 | 300.000 | 300.000 | 350.000 | 290.000 | 300.000 | 300.000 | 300.000 |
| **24h** | 300.000 | <100 | 0 | 0 | 0 | 0 | 0 | 0 |
| **48h** | 145.000 | <100 | 0 | 0 | 0 | 0 | 0 | 0 |
| **4d** | 100.000 | 0 | 0 | 0 | 0 | 0 | 0 | 0 |
| **7d** | 40.000 | 0 | 0 | 0 | 0 | 0 | 0 | 0 |
| **14d** | 500 | 0 | 0 | 0 | 0 | 0 | 0 | 0 |
| **28d** | 0 | 0 | 0 | 0 | 0 | 0 | 0 | 0 |

| **Ps.aeruginosa** | | | | | | | | |
|---|---|---|---|---|---|---|---|---|
| **0'-count** | 290.000 | 290.000 | 290.000 | 360.000 | 270.000 | 290.000 | 290.000 | 290.000 |
| **24h** | 145.000 | <100 | 0 | 2.600 | 0 | 0 | 0 | 0 |
| **48h** | 12.000 | <100 | 0 | 1,0 mill. | 0 | 0 | 0 | 0 |
| **4d** | 0 | 0 | 0 | 17,2 mill. | 0 | 0 | 0 | 0 |
| **7d** | 0 | 0 | 0 | 20,0 mill | 0 | 0 | 0 | 0 |
| **14d** | 0 | 0 | 0 | 15,0 mill | 0 | 0 | 0 | 0 |
| **28d** | 0 | 0 | 0 | 11,8 mill | 0 | 0 | 0 | 0 |

| **SL** | **231001 PE3** | **231001 HE3** | **231001 OC3** | **010801 DE3** | **050601 DEH** | **231001 HE/OC** | **231001 HE/OC/** | **231001 PEHE/OC/DE** |
|---|---|---|---|---|---|---|---|---|
| **Code** | **PE3 Pentandlol 3%** | **HE3 Hexandlol 3%** | **OC3 Octandiol 3%** | **DE 3 Decandtol 3%** | **PE2DE1 Pentandlol 2% Decandlol 1%** | **HE2/OC1 Hexandiol 2% Octandlol 1%** | **PE1HE1O C1 Pentandiol 1% Hexandlol 1% Octandiol 1%** | **HE1OC1D E1 Hexandiol 1% Octandlol 1% Decandiol 1%** |
| Staph. aureus | | | | | | | | |
| 0'-count | 240.000 | 240.000 | 240.000 | 350.000 | 250.000 | 240.000 | 240.000 | 240.000 |
| 24h | 100.000 | <100 | 0 | 3.200 | 100 | 0 | 0 | 0 |
| 48h | 12.000 | <100 | 0 | 0 | 0 | 0 | 0 | 0 |
| 7d | 0 | 0 | 0 | 0 | 0 | 0 | 0 | 0 |
| 14d | 0 | 0 | 0 | 0 | 0 | 0 | 0 | 0 |
| 28d | 0 | 0 | 0 | 0 | 0 | 0 | 0 | 0 |

| C.albi-cans | | | | | | | | |
|---|---|---|---|---|---|---|---|---|
| O'-ccunt | 250.000 | 250.000 | 250.000 | 320.000 | 300.000 | 250.000 | 250.000 | 250.000 |
| 24h | 245.000 | 120.000 | 3.200 | 3.500 | 54.000 | 1.700 | 1.800 | 12.000 |
| 48h | 73.000 | 5.600 | <100 | 0 | 400 | 0 | 0 | <100 |
| 4d | 28.000 | 0 | 0 | 0 | 0 | 0 | 0 | 0 |
| 7d | 23.000 | 0 | 0 | 0 | 0 | 0 | 0 | 0 |
| 14d | <100 | 0 | 0 | 0 | 0 | 0 | 0 | 0 |
| 28d | 0 | 0 | 0 | 0 | 0 | 0 | 0 | 0 |

| A.niger | | | | | | | | |
|---|---|---|---|---|---|---|---|---|
| 0' -count | 260.000 | 260.000 | 260.000 | 250.000 | 250.000 | 260.000 | 260.000 | 260.000 |
| 24h | 180.000 | 120.000 | 55.000 | 73.000 | 180.000 | 12.000 | 30.000 | 180.000 |
| 48h | 180.000 | 120.000 | 6.000 | 160.000 | 82.000 | 10.000 | 18.000 | 160.000 |
| 4d | 180.000 | 120.000 | 6.000 | 100.000 | 64.000 | 8.000 | 16.000 | 140.000 |
| 7d | 140.000 | 80.000 | 1.100 | 100.000 | 20.000 | 600 | 16.000 | 20.000 |
| 14d | 140.000 | 28.000 | 1.000 | 73.000 | 12.000 | 300 | 600 | 6.000 |
| 28d | 100.000 | 800 | 100 | 73.000 | 5.200 | 0 | 0 | 1.000 |

## Patentansprüche

1. Verwendung einer Mischung aus zwei, drei oder mehr unverzweigten 1,2-Alkandiolen, deren Kettenlängen (i) unterschiedlich sind und (ii) jeweils im Bereich von 5 bis 10 C-Atomen liegen, als antimikrobieller Wirkstoff.

2. Verwendung nach Anspruch 1, wobei die Anteile der besagten Diole in der Mischung so eingestellt sind, dass ihre antimikrobielle Wirkung synergistisch verstärkt ist.

3. Verwendung nach Anspruch 1 oder 2, wobei die Mischung 1,2-Hexandiol und ein, zwei oder drei weitere unverzweigte 1,2-Alkandiole umfasst, deren Kettenlänge jeweils im Bereich von 5 bis 10 C-Atomen liegt.

4. Verwendung einer Mischung aus
(a) 1,2-Hexandiol und 1,2-Octandiol,
(b) 1,2-Hexandiol und 1,2-Decandiol,
(c) 1,2-Pentandiol, 1,2-Hexandiol und 1,2-Octandiol,
(d) 1,2-Hexandioi, 1,2-Octandiol und 1,2-Decandiol oder
(e) 1,2-Pentandiol, 1,2-Hexandiol und 1,2-Decandiol
als antimikrobieller Wirkstoff, wobei die Anteile der besagten Diole in der Mischung so eingestellt sind, dass ihre antimikrobielle Wirkung synergistisch verstärkt ist.

5. Verwendung nach einem der Ansprüche 1 bis 4, wobei der Anteil jedes einzelnen Diols im Bereich von 1 bis 99 Gew.-% liegt, vorzugsweise im Bereich von 20 bis 80 Gew.-%, bezogen auf die Gesamtmasse der Mischung der Diole.

6. Verfahren zur kosmetischen und/oder therapeutischen Behandlung von
(a) Körpergeruch verursachenden Mikroorganismen,
(b) Akne verursachenden Mikroorganismen und/oder
(c) Mykosen verursachenden Mikroorganismen,
umfassend die topische Applikation einer antimikrobiell wirksamen Menge eines Gemisches aus zwei, drei oder mehr unverzweigten 1,2-Alkandiolen, deren Kettenlängen (i) unterschiedlich sind und (ii) jeweils im Bereich von 5 bis 10 C-Atomen liegen,
wobei die Anteile der besagten Diole in der Mischung so eingestellt sind, dass ihre antimikrobielle Wirkung synergistisch verstärkt ist.

7. Verwendung einer Mischung aus zwei, drei oder mehr unverzweigten 1,2-Alkandiolen, deren Kettenlängen (i) unterschiedlich sind und (ii) jeweils im Bereich von 5 bis 10 C-Atomen liegen, wobei die Anteile der besagten Diole in der Mischung so eingestellt sind, dass ihre antimikrobiette Wirkung synergistisch verstärkt ist,
(a) zur kosmetischen Behandlung von Körpergeruch verursachenden Mikroorganismen,
(b) zur kosmetischen Behandlung von Akne verursachenden Mikroorganismen,
(c) zur kosmetischen Behandlung von Mykosen verursachenden Mikroorganismen,
(d) zur Behandlung von Mikroorganismen auf oder in unbelebter Materie oder
(e) zur Konservierung eines verderblichen Produktes.

8. Verwendung einer Mischung aus zwei, drei oder mehr unverzweigten 1,2-Alkandiolen, deren Kettenlängen (i) unterschiedlich sind und (ii) jeweils im Bereich von 5 bis 10 C-Atomen liegen, zur Herstellung eines antimikrobiell wirkenden pharmazeutischen Mittels, wobei die Anteile der besagten Diole in der Mischung so eingestellt sind, dass ihre antimikrobielle Wirkung synergistisch verstärkt ist.

9. Verwendung nach einem der Ansprüche 1 - 8, wobei als weiterer Mischungsbestandteil ein antimikrobieller Wirkstoff in einer Menge eingesetzt wird, bei der die antimikrobielle Wirkung des Alkandiolgemisches synergistisch verstärkt wird, wobei der weitere antimikrobielle Wirkstoff kein unverzweigtes 1,2-Alkandiol umfasst.

10. Antimikrobielle Zusammensetzung, umfassend:
(a) als antimikrobiellen Wirkstoff eine Mischung aus zwei, drei oder mehr unverzweigten 1,2-Alkandiolen, deren Kettenlängen (i) unterschiedlich sind und (ii) jeweils im Bereich von 5 bis 10 C-Atomen liegen,
und
(b) eine zu der besagten Mischung kompatible Trägersubstanz sowie gegebenenfalls
(c) einen weiteren antimikrobiellen Wirkstoff, der kein unverzweigtes 1,2 Alkandiol umfasst.
